# EUROPEAN PATENT SPECIFICATION

(11) **EP 1 608 279 B1**
(45) Date of publication and mention of the grant of the patent: **16.09.2009**
(21) Application number: 04704088.6
(22) Date of filing: 21.01.2004
(51) Int. Cl.: A61B 18/18

(54) **A TISSUE ABLATION SYSTEM WITH A SLIDING ABLATING DEVICE**
GEWEBEABLATIONSSYSTEM MIT EINER GLEITENDEN ABLATIONSVORRICHTUNG
SYSTEME D'ABLATION DE TISSU PRESENTANT UN DISPOSITIF D'ABLATION COULISSANT

(30) Priority: 21.01.2003 US 348256
(43) Date of publication of application: 28.12.2005
(73) Proprietor: Maquet Cardiovascular LLC, San Jose, CA 95134 (US)
(72) Inventor: PATIL, Nitin, A., Sunnyvale, CA 94087 (US); AKKAD, Manoj, Livermore, CA 94551 (US); CHIN, Sing, Fatt, Pleasanton CA94566 (US); BERUBE, Dany, Milpitas, CA 95035 (US); MODY, Dinesh, Pleasanton, CA 94588 (US); NORRIS, Nancy, Fremont, CA 94538 (US)
(74) Representative: Browne, Robin Forsythe
(86) International application number: PCT/US2004/001649
(87) International publication number: WO 2004/064613

(56) References cited:
- WO-A-00/56237
- WO-A1-93/15664
- US-A- 5 529 820
- US-A1- 2002 087 151
- US-A1- 2002 128 636
- US-A1- 2003 069 575

## Description

### BACKGROUND OF THE INVENTION

### 1. Field of Invention

The present invention relates, generally, to ablation instrument systems that use ablative energy to ablate internal bodily tissues. More particularly, the present invention relates to preformed guide apparatus which cooperates with energy delivery arrangements to direct the ablative energy in selected directions along the guide apparatus.

### 2. Description of the Prior Art

It is well documented that atrial fibrillation, either alone or as a consequence of other cardiac disease, continues to persist as the most common cardiac arrhythmia. According to recent estimates, more than two million people in the U.S. suffer from this common arrhythmia, roughly 0.15% to 1.0% of the population. Moreover, the prevalence of this cardiac disease increases with age, affecting nearly 8% to 17% of those over 60 years of age.

Atrial arrhythmia may be treated using several methods. Pharmacological treatment of atrial fibrillation, for example, is initially the preferred approach, first to maintain normal sinus rhythm, or secondly to decrease the ventricular response rate. Other forms of treatment include drug therapies, electrical cardioversion, and RF catheter ablation of selected areas determined by mapping. In the more recent past, other surgical procedures have been developed for atrial fibrillation, including left atrial isolation, transvenous catheter or cryosurgical ablation of His bundle, and the Corridor procedure, which have effectively eliminated irregular ventricular rhythm. However, these procedures have for the most part failed to restore normal cardiac hemodynamics, or alleviate the patient's vulnerability to thromboembolism because the atria are allowed to continue to fibrillate. Accordingly, a more effective surgical treatment was required to cure medically refractory atrial fibrillation of the Heart.

On the basis of electrophysiologic mapping of the atria and identification of macroreentrant circuits, a surgical approach was developed which effectively creates an electrical maze in the atrium (i.e., the MAZE procedure) and precludes the ability of the atria to fibrillate. Briefly, in the procedure commonly referred to as the MAZE III procedure, strategic atrial incisions are performed to prevent atrial reentry circuits and allow sinus impulses to activate the entire atrial myocardium, thereby preserving atrial transport function postoperatively. Since atrial fibrillation is characterized by the presence of multiple macroreentrant circuits that are fleeting in nature and can occur anywhere in the atria, it is prudent to interrupt all of the potential pathways for atrial macroreentrant circuits. These circuits, incidentally, have been identified by intraoperative mapping both experimentally and clinically in patients.

Generally, this procedure includes the excision of both atrial appendages, and the electrical isolation of the pulmonary veins. Further, strategically placed atrial incisions not only interrupt the conduction routes of the common reentrant circuits, but they also direct the sinus impulse from the sinoatrial node to the atrioventricular node along a specified route. In essence, the entire atrial myocardium, with the exception of the atrial appendages and the pulmonary veins, is electrically activated by providing for multiple blind alleys off the main conduction route between the sinoatrial node to the atrioventricular node. Atrial transport function is thus preserved postoperatively as generally set forth in the series of articles: Cox, Schuessler, Boineau, Canavan, Cain, Lindsay, Stone, Smith, Corr, Change, and D'Agostino, Jr., The Surgical Treatment Atrial Fibrillation (pts. 1-4), 101 THORAC CARDIOVASC SURG., 402-426, 569-592 (1991).

While this MAZE III procedure has proven effective in ablating medically refractory atrial fibrillation and associated detrimental sequelae, this operational procedure is traumatic to the patient since this is an open-heart procedure and substantial incisions are introduced into the interior chambers of the Heart. Consequently, other techniques have been developed to interrupt atrial fibrillation restore sinus rhythm. One such technique is strategic ablation of the atrial tissues through ablation catheters.

Most approved ablation catheter systems now utilize radio frequency (RF) energy as the ablating energy source. Accordingly, a variety of RF based catheters and power supplies are currently available to electrophysiologists. However, radio frequency energy has several limitations including the rapid dissipation of energy in surface tissues resulting in shallow "burns" and failure to access deeper arrhythmic tissues. Another limitation of RF ablation catheters is the risk of clot formation on the energy emitting electrodes. Such clots have an associated danger of causing potentially lethal strokes in the event that a clot is dislodged from the catheter. It is also very difficult to create continuous long lesions with RF ablation instruments.

As such, catheters which utilize other energy sources as the ablation energy source, for example in the microwave frequency range, are currently being developed. Microwave frequency energy, for example, has long been recognized as an effective energy source for heating biological tissues and has seen use in such hyperthermia applications as cancer treatment and preheating of blood prior to infusions. Accordingly, in view of the drawbacks of the traditional catheter ablation techniques, there has recently been a great deal of interest in using microwave energy as an ablation energy source. The advantage of microwave energy is that it is much easier to control and safer than direct current applications and it is capable of generating substantially larger and longer lesions than RF catheters, which greatly simplifies the actual ablation procedures. Such microwave ablation systems are described in the U.S. Patent Numbers 4,641,649 to Walinsky; 5,246,438 to Langberg; 5,405,346 to Grundy, et al.; and 5,314,466 to Stern, et al.

Most of the existing microwave ablation catheters contemplated the use of longitudinally extending helical antenna coils that direct the electromagnetic energy in all radial directions that are generally perpendicular to the longitudinal Axis of the catheter. Although such catheter designs work well for a number of applications, such radial output is inappropriate when the energy needs to be directed toward the tissue to ablate only.

Consequently, microwave ablation instruments have recently been developed which incorporate microwave antennas having directional reflectors. Typically, a tapered directional reflector is positioned peripherally around the microwave antenna to direct the waves toward and out of a window portion of the antenna assembly. These ablation instruments, thus, are capable of effectively transmitting electromagnetic energy in a more specific direction. For example, the electromagnetic energy may be transmitted generally perpendicular to the longitudinal axis of the catheter but constrained to a selected radial region of the antenna, or directly out the distal end of the instrument. Typical of these designs are described in the U.S. Patent Nos 6245062 and 6287302.
US 2002/0087151 discloses a system for ablating tissue within a body of a patient, comprising a handle portion having proximal and distal ends; a flexible elongated tubular member having at least one lumen therethrough operably attached to the distal end of the handle portion; an energy delivery device comprising at least one ablative element, the energy delivery device operably attached to the handle portion and adapted to translate within a first of the at least on lumen of the tubular member; and an ablative energy source operably attached to the at least one ablative energy element.

In these designs, the resonance frequency of the microwave antenna is preferably tuned assuming contact between the targeted tissue or blood and a contact region of the antenna assembly extending longitudinally adjacent to the antenna longitudinal axis. Hence, should a portion of, or substantially all of, the exposed contact region of the antenna not be in contact with the targeted tissue or blood during ablation, the resonance frequency will be adversely changed and the antenna will be untuned. As a result, the portion of the antenna not in contact with the targeted tissue or blood will radiate the electromagnetic radiation into the surrounding air. The efficiency of the energy delivery into the tissue will consequently decrease which in turn causes the penetration depth of the lesion to decrease.

This is particularly problematic when the microwave antenna is not in the blood pool, or when the tissue surfaces are substantially curvilinear, or when the targeted tissue for ablation is difficult to access, such as in the interior chambers of the Heart. Since these antenna designs are generally relatively rigid, it is often difficult to maneuver substantially all of the exposed contact region of the antenna into abutting contact against the targeted tissue. In these instances, several ablation instruments, having antennas of varying length and shape, may be necessary to complete just one series of ablations.

### SUMMARY OF THE INVENTION

The invention is as defined in claim 1.
Accordingly, a system for ablating a selected portion of a contact surface of biological tissue is provided. The system is particularly suitable to ablate cardiac tissue, and includes an elongated ablation sheath having a preformed shape adapted to substantially conform a predetermined surface thereof with the contact surface of the tissue. The ablation sheath defines an ablation lumen extending therethrough along an ablation path proximate to the predetermined surface. An elongated ablative device includes a flexible ablation element which cooperate with an ablative energy source which is sufficiently strong for tissue ablation. The ablative device is formed and dimensioned for longitudinal sliding receipt through the ablation lumen of the ablation sheath for selective placement of the ablative device along the ablation path created by the ablation sheath. The ablation lumen and the ablative device cooperate to position the ablative device proximate to the ablation sheath predetermined surface for selective ablation of the selected portion.

Accordingly, the ablation sheath in its preshaped form functions as a guide device to guide the ablative device along the ablation path when the predetermined surface of the ablation sheath properly contacts the biological tissue. Further, the cooperation between the ablative device and the ablation lumen, as the ablative device is advanced through the lumen, positions the ablative device in a proper orientation to facilitate ablation of the targeted tissue during the advancement. Thus, once the ablation sheath is stationed relative the targeted contact surface, the ablative device can be easily advanced along the ablation path to generate the desired tissue ablations.

In one embodiment, the ablative device is a microwave antenna assembly which includes a flexible shield device coupled to the antenna substantially shield a surrounding area of the antenna from the electromagnetic field radially generated therefrom while permitting a majority of the field to be directed generally in a predetermined direction toward the ablation sheath predetermined surface.. The microwave antenna assembly further includes a flexible insulator disposed between the shield device and the antenna. A window portion of the insulator is defined which enables transmission of the directed electromagnetic field in the predetermined direction toward the ablation sheath predetermined surface. The antenna, the shield device and the insulator are formed for manipulative bending thereof, as a unit, to one of a plurality of contact positions to generally conform the window portion to the ablation sheath predetermined surface as the insulator and antenna are advanced through the ablation lumen.

In another embodiment, to facilitate alignment of the ablative device assembly in the ablation lumen, the ablative device provides a key device which is slideably received in a mating slot portion of the ablation lumen. In still another embodiment, the system includes a guide sheath defining a guide lumen formed and dimensioned for sliding receipt of the ablation sheath therethrough. The guide sheath is pre-shaped to facilitate positioning of the ablation sheath toward the selected portion of the contact surface when the ablation sheath is advanced through guide lumen.

The ablation sheath includes a bendable shape retaining member extending longitudinally therethrough which is adapted to retain the preformed shape of the ablation sheath once positioned out of the guide lumen of the guide sheath.

The ablative energy is preferably provided by a microwave ablative device. Other suitable tissue ablation devices, however, include cryogenic, ultrasonic, laser and radiofrequency, to name a few.

The system of the present invention may be used in a method for treatment of a Heart includes forming a penetration through a muscular wall of the Heart into an interior chamber thereof; and positioning a distal end of an elongated ablation sheath through the penetration. The ablation sheath defines an ablation lumen extending along an ablation path therethrough. The method further includes contacting, or bringing close enough, a predetermined surface of the elongated ablation sheath with a first selected portion of an interior surface of the muscular wall; and passing a flexible ablative device through the ablation lumen of the ablation sheath for selective placement of the ablative device along the ablation path. Once these events have been performed, the method includes applying the ablative energy, using the ablative device and the ablation energy source, which is sufficiently strong to cause tissue ablation.

In one embodiment, the passing is performed by incrementally advancing the ablative device along a plurality of positions of the ablation path to produce a substantially continuous lesion. Before the positioning event, the method includes placing a distal end of a guide sheath through the penetration, and then positioning the distal end of the ablation sheath through the guide lumen of the guide sheath.

In still another embodiment, before the placing event, piercing the muscular wall with a piercing sheath. The piercing sheath defines a positioning passage extending therethrough, The placing the distal end of a guide sheath is performed by placing the guide sheath distal end through the positioning passage of the piercing sheath.

In yet another configuration, the positioning of the distal end event includes advancing the ablation sheath toward the first selected portion of the interior surface of the muscular wall through a manipulation device extending through a second penetration into the Heart interior chamber independent from the first named penetration.

In another embodiment, a system for ablating tissue within a body of a patient is provided including an elongated rail device and an ablative device. The rail device is adapted to be positioned proximate and adjacent to a selected tissue region to be ablated within the body of the patient. The ablative device includes a receiving passage configured to slideably receive the rail device longitudinally therethrough. This enables the ablative device to be slideably positioned along the rail substantially adjacent to or in contact with the selected tissue region. The ablative device, having an energy delivery portion which is adapted to be coupled to an ablative energy source, can then be operated to ablate the selected tissue region.

In this configuration, the ablative device is adapted to directionally emit the ablative energy from the energy delivery portion. A key assembly cooperates between the ablative device and the rail member, thus, to properly align the directionally emitted ablative energy toward the tissue region to be ablated. This primarily performed by providing a rail device with a non-circular transverse cross-sectional dimension. The receiving passage of the ablative device further includes a substantially similarly shaped non-circular transverse cross-sectional dimension to enable sliding of the ablative device in a manner continuously aligning the directionally emitted ablative energy toward the tissue region to be ablated as the ablative device advances along the rail device.

According to the invention, the ablation sheath comprises at least one non-permeable portion, preventing ingress of fluids, including liquids and/or gases, from a point external to the ablation sheath to the internal location of the ablative device.

### BRIEF DESCRIPTION OF THE DRAWINGS

The assembly of the present invention has other objects and features of advantage which will be more readily apparent from the following description of the best mode of carrying out the invention and the appended claims, when taken in conjunction with the accompanying drawing, in which:

FIGURES 1A and 1B are fragmentary, top perspective views, partially broken-away, of the ablation system constructed in accordance with the present invention, and illustrating advancement of a bendable directional reflective microwave antenna assembly through an ablation lumen of a ablation sheath.

FIGURES 2A-2D is series of fragmentary, side elevation views, in partial cross-section, of the Heart, and illustrating advancement of the ablation system of present invention into the left atrium for ablation of the targeted tissue.

FIGURE 3 is a fragmentary, side elevation view, in partial cross-section, of the Heart showing a pattern of ablation lesions to treat atrial fibrillation.

FIGURES 4A and 4B are a series of enlarged, fragmentary, top perspective view of a pigtail ablation sheath of the ablation system of FIGURES 2C and 2D, and exemplifying the ablation sheath being advanced into one of the pulmonary vein orifices.

FIGURE 5 is a front schematic view of a patient's cardiovascular system illustrating the positioning of a transseptal piercing sheath through the septum wall of the patient's Heart.

FIGURE 6 is a fragmentary, side elevation view, in partial cross-section, of another embodiment of the ablation sheath of the present invention employed for lesion formation.

FIGURE 7 is a fragmentary, side elevation view, in partial cross-section, of yet another embodiment of the ablation sheath of the present invention employed for another lesion formation.

FIGURE 8 is an enlarged, front elevation view, in cross-section, of the ablation system of FIGURE 1 positioned through the trans-septal piercing sheath.

FIGURE 9 is an enlarged, front elevation view, in cross-section, of the ablation sheath and the antenna assembly of the ablation system in FIGURE 8 contacting the targeted tissue.

FIGURE 10 is an enlarged, front elevation view, in cross-section, of the antenna assembly taken substantially along the plane of the line 10-10 in FIGURE 9.

FIGURE 11 is a diagrammatic top plan view of an alternative embodiment microwave ablation instrument system constructed in accordance with one embodiment of the present invention.

FIGURE 12 is an enlarged, fragmentary, top perspective view of the ablation instrument system of FIGURE 11, illustrated in a bent position to conform the ablation sheath to a surface of the tissue to be ablated.

FIGURES 13A-13D is a series of side elevation views, in cross-section, of the ablation sheath of the present invention illustrating advancement of the ablation device incrementally through the ablation sheath to form plurality of overlapping lesions.

FIGURE 14A is a fragmentary, side elevation view of a laser-type ablation device of the present invention.

FIGURE 14B is a front elevation view of the laser-type energy delivery portion taken along the plane of the line 14B-14B in FIGURE 14A.

FIGURE 15A is a fragmentary, side elevation view of a cryogenic-type ablation device of the present invention.

FIGURE 15B is a front elevation view of the cryogenic-type energy delivery portion taken along the plane of the line 15B-15B in FIGURE 15A.

FIGURE 16 is a fragmentary, side elevation view, in cross-section, of an ultrasonic-type ablation device of the present invention.

FIGURE 17 is an enlarged, fragmentary, top perspective view of an alternative embodiment ablation sheath having an opened window portion.

FIGURE 18 is a fragmentary, side elevation view of an alternative embodiment ablation assembly employing a rail system.

FIGURE 19 is a front elevation view of the energy delivery portion of the ablation rail system taken along the plane of the line 19-19 in FIGURE 18.

FIGURES 20A-20C are cross-sectional views of alternative key systems in accordance with the present invention.

FIGURE 21 is a fragmentary, diagrammatic, front elevation view of a torso applying one embodiment of the present invention through a minimally invasive technique.

FIGURE 22 is a top plan view, in cross-section of the fragmentary, diagrammatic, top plan view of the torso of FIGURE 21 applying the minimally invasive technique.

FIGURE 23 is a perspective view of an alternative embodiment of an ablation system in accordance with the present invention.

FIGURE 24A is an enlarged, front elevation view, in cross-section, of the ablation sheath of the ablation system in FIGURE 23.

FIGURE 24B is an enlarged, from elevation view, in cross-section, of the antenna assembly of the ablation system in FIGURE 23.

FIGURE 24C is a side elevation view, in partial cross-section, of the antenna assembly of the ablation system in FIGURE 23.

FIGURE 25 is a side elevation view, in partial cross-section, of the handle portion of the ablation system in FIGURE 23. FIGURE 26 depicts an exemplary placement of the ablation sheath.

FIGURE 26A is a partial sectional side view of the ablation sheath depicting the porous nature of the sheath, according to one embodiment of the invention.

FIGURES 26B-C are partial sectional side views of the ablation sheath of FIGURE 26A adapted to include a non-permeable portion.

### DETAILED DESCRIPTION OF THE INVENTION

While the present invention will be described with reference to a few specific embodiments, the description is illustrative of the invention and is not to be construed as limiting the invention. Various modifications to the present invention can be made to the preferred embodiments by those skilled in the art without departing from the scope of the invention as defined by the appended claims. It will be noted here that for a better understanding, like components are designated by like reference numerals throughout the various Figures.

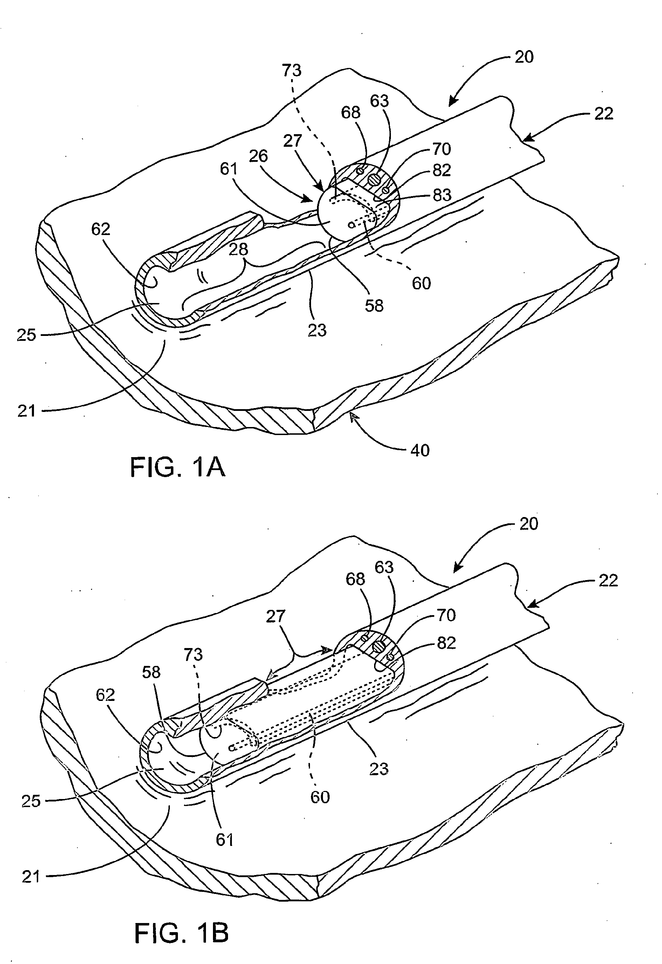

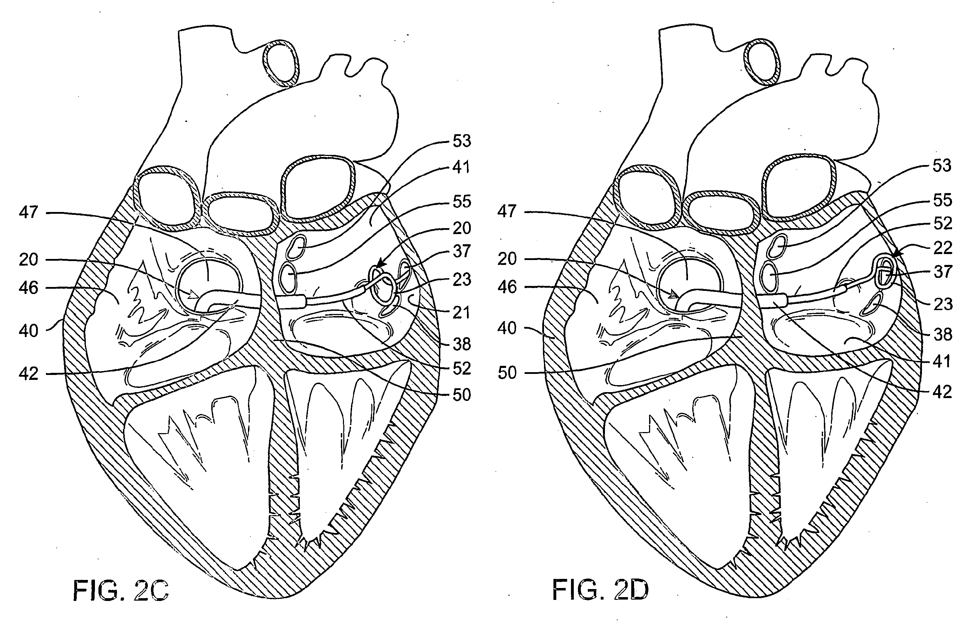

Turning generally now to FIGURES 1A-2D, an ablation system, generally designated 20, is provided for transmurality ablating a targeted tissue 21 of biological tissue. The system 20 is particularly suitable to ablate the epicardial or endocardial tissue 40 of the heart, and more particularly, to treat medically refractory atrial fibrillation of the Heart. The ablation system 20 for ablating tissue within a body of a patient includes an elongated flexible tubular member 22 having at least one lumen 25 (FIGURES 1A, 1B, 8 and 9) and including a pre-shaped distal end portion (E.g., FIGURE 2C, 6 and 7) which is shaped to be positioned adjacent to or in contact with a selected tissue region 21 within the body of the patient. An ablative device, generally designated 26, is configured to be slideably received longitudinally within the at least one lumen 25, and includes an energy delivery portion 27 located near a distal end portion of the ablative device 26 which is adapter to be coupled to an ablative energy source (not shown).

The ablative device is preferably provided by a microwave ablation device 26 formed to emit microwave energy sufficient to cause tissue ablation. As will be described in greater detail below, however, the ablative device energy may be provided by a laser ablation device, a Radio Frequency (RF) ablation device, an ultrasound ablation device or a cryoablation device.

The tubular member 22 is in the form of an elongated ablation sheath having a resiliently preformed shape adapted to substantially conform a predetermined contact surface 23 of the sheath with the targeted tissue region 21. In another embodiment, the ablation sheath is malleable. Yet, in another embodiment, the ablation sheath is flexible. The lumen 25 of the tubular member extends therethrough along an ablation path proximate to the predetermined contact surface. Preferably, as will be described in more detail below, the ablative device 26 includes a flexible energy delivery portion 27 selectively generating an electromagnetic field which is sufficiently strong for tissue ablation. The energy delivery portion 27 is formed and dimensioned for longitudinal sliding receipt through the ablation lumen 25 of the ablation sheath 22 for selective placement of the energy delivery portion along the ablation path. The ablation lumen 25 and the ablative device 26 cooperate to position the energy delivery portion 27 proximate to the ablation sheath 22 predetermined contact surface 23 of the sheath for selective transmural ablation of the targeted tissue 21 within the electromagnetic field when the contact surface 23 strategically contacts or is positioned close enough to the targeted tissue 21.

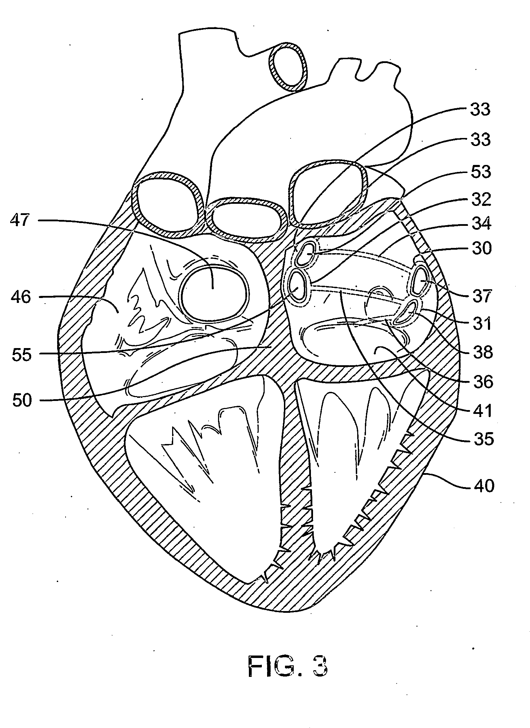

Accordingly, in one preferred embodiment, the pre-shaped ablation sheath 22 functions to unidirectionally guide or position the energy delivery portion 27 of the ablative device 26 properly along the predetermined ablation path 28 proximate to the targeted tissue region 21 as the energy delivery portion 27 is advanced through the ablation lumen 25. By positioning the energy delivery portion 27, which is preferably adapted to emit a directional ablation field, at one of a plurality of positions incrementally along the ablation path (FIGURES 1A and 1B) in the lumen 25, a single continuous or plurality of spaced-apart lesions can be formed. In other instances, the antenna length may be sufficient to extend along the entire ablation path 28 so that only a single ablation sequence is necessary. While the apparatus of the present invention is applicable to ablate any biological tissue which requires the formation of controlled lesions (as will be described in greater detail below), this ablation system is particularly suitable for ablating endocardial or epicardial tissue of the Heart. For example, the present invention may be applied in an intra-coronary configuration where the ablation procedure is performed on the endocardium of any cardiac chamber. Specifically, such ablations may be performed on the isthmus to address atrial flutter, or around the pulmonary vein ostium, electrically isolating the pulmonary veins, to treat medically refractory atrial fibrillation (FIGURE 3). This procedure requires the precise formation of strategically placed endocardial lesions 30-36 which collectively isolate the targeted regions. By way of example, any of the pulmonary veins may be collectively isolated to treat chronic atrial fibrillation. The annular lesion isolating one or more than one pulmonary vein can be linked with another linear lesion joining the mitral valve annulus. In another example, the annular lesion isolating one or more than one pulmonary vein can be linked with another linear lesion joining the left atrium appendage.

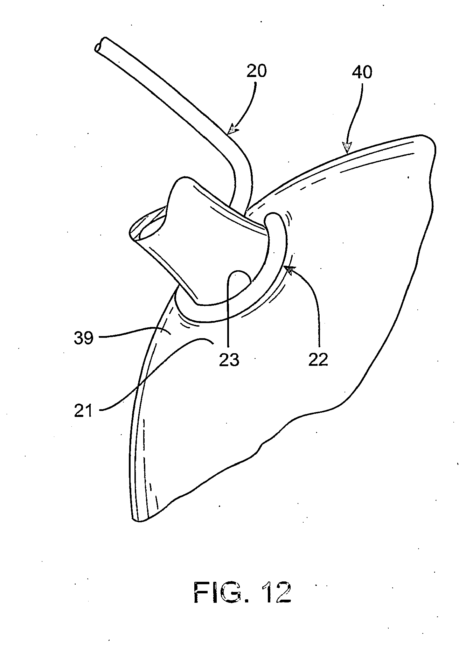

In a preferred embodiment, the pre-shaped ablation sheath 22 and the sliding ablative device 26 may applied to ablate the epicardial tissue 39 of the Heart 40 as well (FIGURE 12). An annular ablation, for instance, may be formed around the pulmonary vein for electrical isolation from the left atrium. As another example, the lesions may be created along the transverse sinus and oblique sinus as part of the collective ablation pattern to treat atrial fibrillation for example.

The application of the present invention, moreover, is preferably performed through minimally invasive techniques. It will be appreciated, however, that the present invention may be applied through open chest techniques as well.

Briefly, to illustrate the operation of the present invention, a flexible pre-shaped tubular member (i.e., ablation sheath 22) in the form of a pigtail is shown in FIGURES 2C and 2d which is specifically configured to electrically isolate a pulmonary vein of the Heart 40. The isolating lesions are preferably made on the posterior wall of the left atrium, around the ostium of one, or more than one of a pulmonary vein.

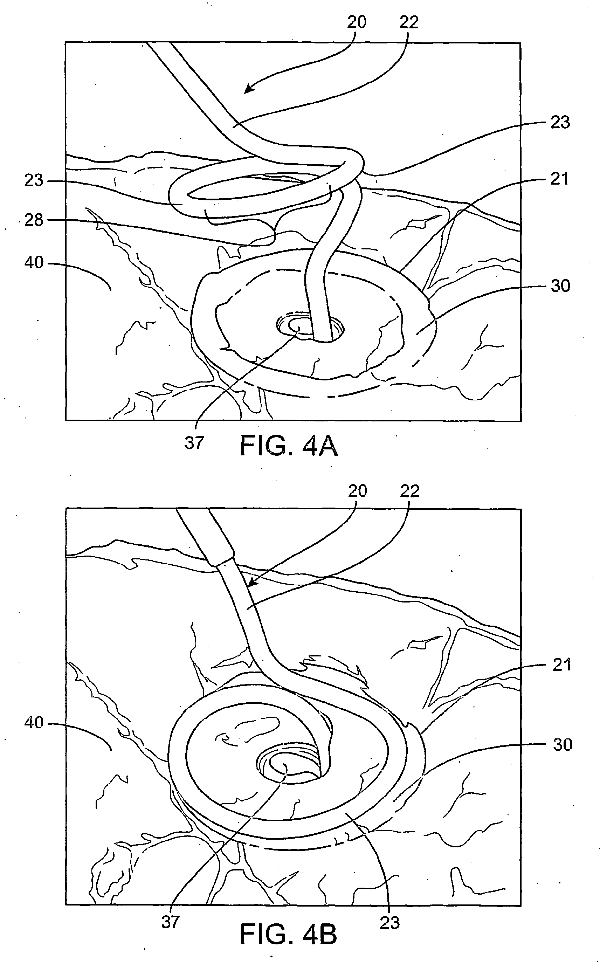

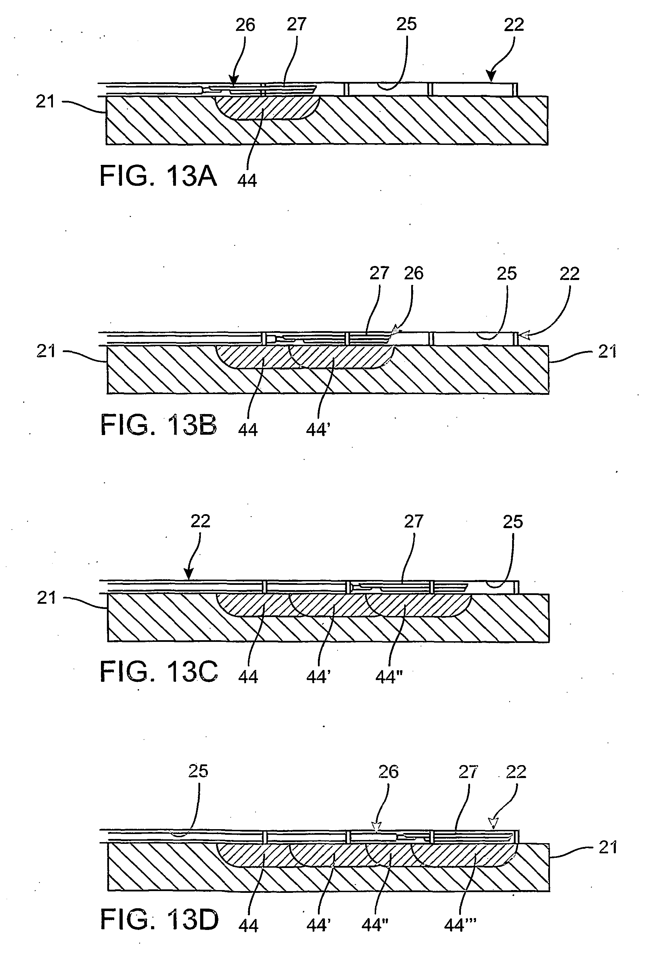

In this example and as illustrated in FIGURES 4A and 4B, a distal end of the pigtail-shaped ablation sheath or tubular member 22 is positioned into the left superior pulmonary vein orifice 37 from the left atrium 41. As the ablation sheath 22 is further advanced, a predetermined contact surface 23 of the ablation sheath is urged adjacent to or into contact with the endocardial surface of the targeted tissue region 21 (FIGURES 2D and 4B). Once the ablation sheath 22 is properly positioned and oriented, the ablative device 26 is advanced through the ablation lumen 25 of the ablation sheath 22 (FIGURES 1A and 1B) which moves the energy delivery portion 27 of the ablative device along the ablation path. When the energy delivery portion 27 is properly oriented and positioned in the ablation lumen 25, the directional ablation field may be generated to incrementally ablate (FIGURES 13A-13D) the epicardial surface of the targeted tissue 21 along the ablation path to isolate the Left Superior Pulmonary Vein (LIPV)

Accordingly, as shown in FIGURES 13A-13D, as the energy delivery portion 27 is incrementally advanced through the lumen 25, overlapping lesion sections 44-44"' are formed by the ablation field which is directional in one preferred embodiment. Collectively, a continuous lesion or series of lesions can be formed which essentially three-dimensionally "mirror" the shape of the contact surface 23 of the ablation sheath 22 which is positioned adjacent to or in contact with the targeted tissue region. These transmural lesions may thus be formed in any shape on the targeted tissue region such as rectilinear, curvilinear or circular in shape. Further, depending upon the desired ablation lines pattern, both opened and closed path formation can be constructed.

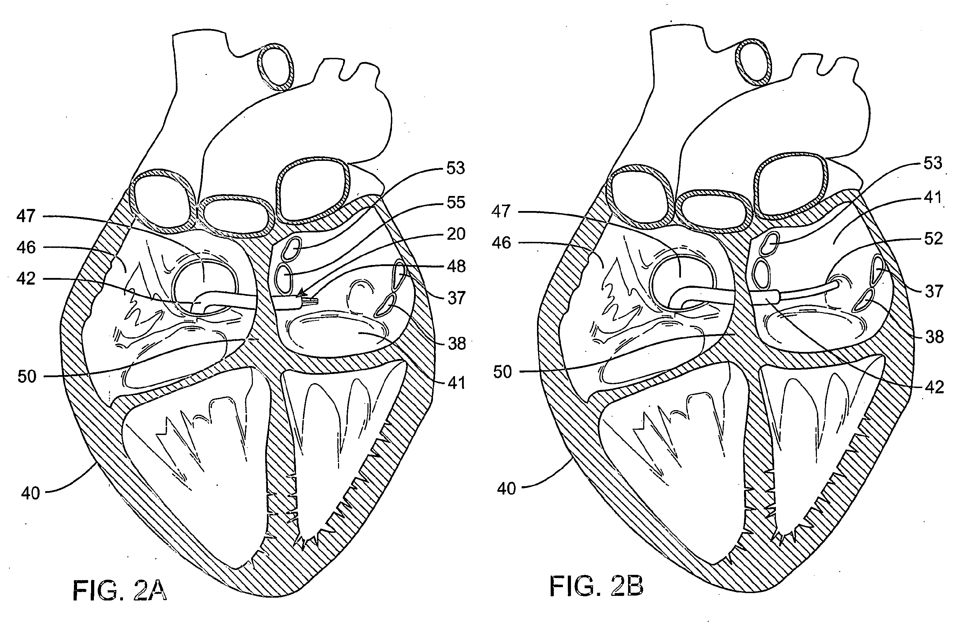

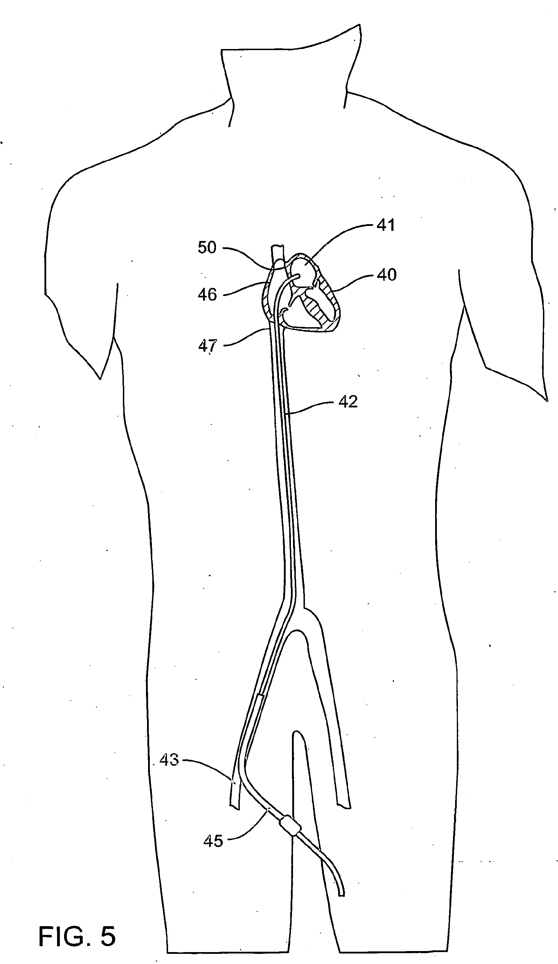

Referring now to FIGURES 2A, 2D and 5, a minimal invasive application of the present invention is illustrated for use in ablating Heart tissue. By way of example, a conventional transseptal piercing sheath 42 is introduced into the femoral vein 43 through a venous cannula 45 (FIGURE 5). The piercing sheath is then intravenously advanced into the right atrium 46 of the Heart 40 through the inferior vena cava orifice 47. These piercing sheaths are generally resiliently pre-shaped to direct a conventional piercing device 48 toward the septum wall 50. The piercing device 48 and the piercing sheath 42 are manipulatively oriented and further advanced to pierce through the Septum wall 50, as a unit, of access into the left atrium 41 of the Heart 40 (FIGURE 2A).

These conventional devices are commonly employed in the industry for accessing the left atrium or ventricle, and have an outer diameter in the range of about 4mm (0.16 inch) to about 4.4 mm (0.175 inch), while having an inner diameter in the range of about 2.2mm (0.09 inch) to about 3.4mm (0.135 inch).

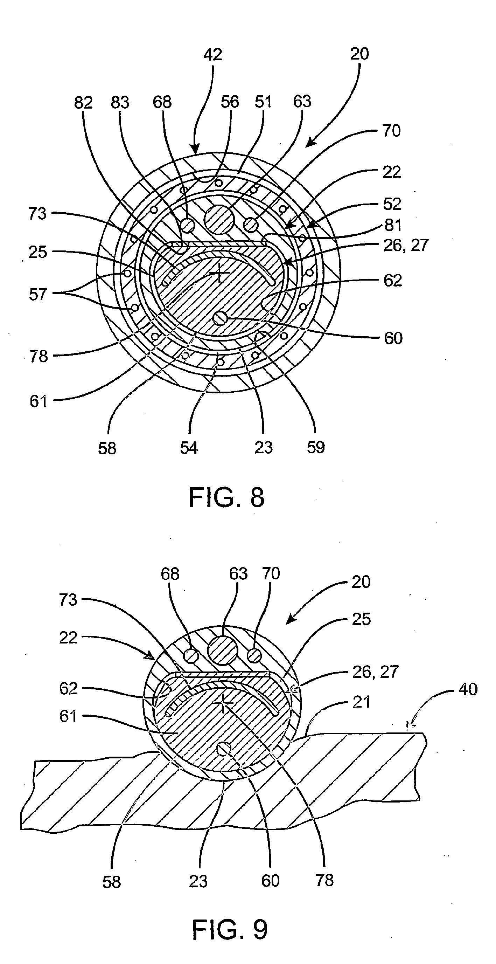

Once the piercing device is withdrawn from a positioning passage 51 (FIGURE 8) of the piercing sheath 42, a guide sheath 52 of the ablation system 20 is slideably advanced through the positioning passage and into a cardiac chamber such as the left atrium 41 thereof (FIGURE 2B). The guide sheath 52 is essentially a pre-shaped, open-ended tubular member which is inserted into the coronary circulation to direct and guide the advancing ablation sheath 22 into a selected cardiac chamber (i.e., the left atrium, right atrium, left ventricle or right ventricle) and toward the general direction of the targeted tissue. Thus, the guide sheath 52 and the ablation sheath 22 telescopically cooperate to position the predetermined contact surface 23 thereof substantially adjacent to or in contact with the targeted tissue region.

Moreover, the guide sheath and the ablation sheath cooperate to increase the structural stability of the system as the ablation sheath is rotated and manipulated from its proximal end into ablative contact with the targeted tissue 21 (FIGURE 2A). As the distal curved portions of the ablation sheath 22, which is inherently longer than the guide sheath, is advanced past the distal lumen opening of the guide sheath, these resilient curved portions will retain their original unrestrained shape.

The telescopic effect of these two sheaths is used to position the contact surface 23 of the ablation sheath 22 substantially adjacent to or in contact with the targeted tissue. Thus, depending upon the desired lesion formation, the same guide sheath 52 may be employed for several different procedures. For example, the lesion 30 encircling the left superior pulmonary vein ostium and the Left Inferior Pulmonary Vein Ostium (RIPVO) lesion 31 (FIGURE 3) may be formed through the cooperation of the pigtail ablation sheath 22 and the same guide sheath 52 of FIGURE 2B and 2D, while the same guide sheath may also be utilized with a different ablation sheath 22 (FIGURE 4) to create the long linear lesion 34 as shown in FIGURE 3.

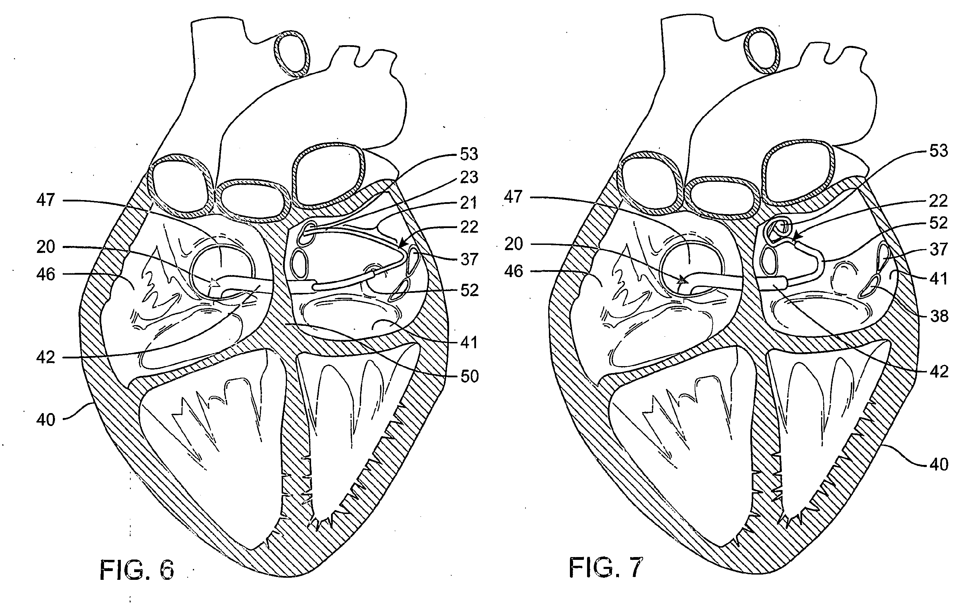

In contrast, as illustrated in FIGURE 7, another guide sheath 52 having a different pre-shaped distal end section may be applied to direct the advancing ablation sheath 22 back toward the in the left and right superior pulmonary vein orifices 53, 55. Thus, several pre-shaped guide sheaths, and the corresponding ablation sheaths, as will be described, cooperate to create a predetermined pattern of lesions (E.g., a MAZE procedure) on the tissue.

In the preferred embodiment, the guide sheath 52 is composed of a flexible material which resiliently retains its designated shape once external forces urged upon the sheath are removed. These external forces, for instance, are the restraining forces caused by the interior walls 56 of the transseptal piercing sheath 42 as the guide sheath 52 is advanced or retracted therethrough. While the guide sheath 52 is flexible, it must be sufficiently rigid so as to substantially retain its original unrestrained shape, and not to be adversely influenced by the Ablation sheath 22, as the ablation sheath is advanced through the lumen of the guide sheath. Such flexible, biocompatible materials may be composed of braided Pebax or the like having an outer diameter formed and dimensioned for sliding receipt longitudinally through the positioning passage 51 of the transseptal piercing sheath 42. The outer dimension is therefore preferably cylindrical having an outer diameter in the range of about 2.2mm (0.09 inch) to about 3.7mm (0.145 inch) and more preferably about 3.4 mm (0.135 inch), while having an inner diameter in the range of about 1.3 mm (0.05 inch) to about 3.2 mm (0.125 inch), and more preferably about 2.9 mm (0.115 inch). This cylindrical dimension enables longitudinal sliding receipt, as well as axial rotation, in the positioning passage 51 to properly place and advance the guide sheath 52. Thus, the dimensional tolerance between the cylindrical-shaped, outer peripheral wall of the guide sheath 52 and the interior walls 56 of the transseptal piercing sheath 42 should be sufficiently large to enable reciprocal movement and relative axial rotation therebetween, while being sufficiently small to substantially prevent lateral displacement therebetween as the ablation sheath 22 is urged into contact with the targeted tissue 21. For example, the dimensional tolerance between the transverse cross-sectional periphery of the interior walls 56 of the positioning passage 51 and that of the substantially conforming guide sheath 52 should be in the range of about 0.13mm (0.005 inches) to about 0.50 mm (0.020 inches).

To increase the structural integrity of the guide sheath 52, metallic braids 57 are preferably incorporated throughout the sheath when the guide sheath is molded to its preformed shape. These braids 57 are preferably provided by 0.05 mm (0.002 inches) wires composed of 304 stainless steel evenly spaced about the sheath.

Once the guide sheath 52 is properly positioned and oriented relative the transseptal sheath 42, the ablation sheath 22 is advanced through a guide lumen 54 (FIGURE 8) of the guide sheath 52 toward the targeted tissue. Similar to the pre-shaped guide sheath 52, the ablation sheath 22 is pre-shaped in the form of the desired lesions to be formed in the endocardial surface of the targeted tissue 21. As best viewed in FIGURES 2D, 6 and 7, each ablation sheath 52 is adapted facilitate an ablation in the targeted tissue 21 generally in the shape thereof. Thus, several pre-shaped ablation sheaths cooperate to form a type of steering system to position the ablation device about the targeted tissue. Collectively, a predetermined pattern of linear and curvilinear lesions (e.g. a MAZE procedure) can be ablated on the targeted tissue region.

Again, similar to the guide sheath 52, the ablation sheath 22 is composed of a flexible material which resiliently retains its designated shape once external forces urged upon the sheath are removed. These external forces, for instance, are the restraining forces caused by the interior walls 59 defining the guide lumen 54 of the guide sheath 52 as the ablation sheath 22 is advanced or retracted therethrough. Such flexible, biocompatible materials may be composed of Pebax or the like having an outer diameter formed and dimensioned for sliding receipt longitudinally through the guide lumen 54 of the ablation sheath 22. As mentioned, the inner diameter of the guide lumen 54 is preferably in the range of about 1.3 mm (0.050 inch) to about 3.2 mm (0.125 inch), and more preferably about 2.9 mm (0.115 inches), while the ablation sheath 26 has an outer diameter in the range of about 10.1 mm (0.40 inch) to about 2.9 mm (0.115 inch), and more preferably about 2.7 mm (0.105 inch).

The concentric cylindrical dimensions enable longitudinal sliding receipt, as well as axial rotation, of the ablation sheath 22 in the guide lumen 54 to properly place and advance the it toward the targeted tissue 21. Thus, the dimensional tolerance between the cylindrical-shaped, outer peripheral wall of the ablation sheath 22 and the interior walls 59 of the guide lumen 54 of the guide sheath 52 should be sufficiently large to enable reciprocal movement and relative axial rotation therebetween, while being sufficiently small to substantially prevent lateral displacement therebetween as the ablation sheath 22 is urged into contact with the targeted tissue 21. For example, the dimensional tolerance between the transverse cross-sectional periphery of the guide lumen 54 and that of the substantially conforming energy delivery portion 27 should be in the range of about 0.02 mm (0.001 inches) to about 0.13 mm (0.005 inches).

As above-indicated, the pre-shaped ablation sheath 22 facilitates guidance of the ablative device 26 along the predetermined ablation path 28. This is primarily performed by advancing the energy delivery portion 27 of the ablative device 26 through the ablation lumen 25 of the ablation sheath 22 which is preferably off-set from the longitudinal axis 78 thereof. As best viewed in FIGURES 8 and 9, this off-set positions the energy delivery portion 27 relatively closer to the predetermined contact surface 23 of the ablation sheath 22, and hence the targeted tissue 21. Moreover, when using directional fields such as those emitted from their energy delivery portion 27, it is important to provide a mechanism for continuously aligning the directional field of the energy delivery portion 27 with the tissue 21 targeted for ablation. Thus, in this design, the directional field must be continuously aligned with the predetermined contact surface 23 of the ablation sheath 22 as the energy delivery portion 27 is advanced through the ablation lumen 25 since the ablation sheath contact surface 23 is designated to contact or be close enough to the targeted tissue.

If the directional field is not aligned correctly, for example, the energy may be transmitted into surrounding fluids and tissues designated for preservation rather than into the targeted tissue region. Therefore, in accordance with another aspect of the present invention, a key structure 48 (FIGURES 1, 8 and 9) cooperates between the ablative device 26 and the ablation lumen 25 to orient the directive energy delivery portion 27 of the ablative device continuously toward the targeted tissue region 21 as it is advanced through the lumen. This key structure 48, thus, only allows receipt of the energy delivery portion 27 in the lumen in one orientation. More particularly, the key structure 48 continuously aligns a window portion 58 of the energy delivery portion 27 substantially adjacent the predetermined contact surface 23 of the ablation sheath 22 during advancement. This window portion 58, as will be described below, enables the transmission of the directed ablative energy from the energy delivery portion 27, through the contact surface 23 of the ablation sheath 22 and into the targeted tissue region. Consequently, the directional ablative energy emitted from the energy delivery portion will always be aligned with the contact surface 23 of the ablation sheath 22, which is positioned adjacent to or in contact with the targeted tissue region 21, to maximize ablation efficiency. By comparison, the ablation sheath 22 is capable of relatively free rotational movement axially in the guide lumen 54 of the guide sheath 52 for maneuverability and positioning of the ablation sheath therein.

As mentioned, the transverse cross-sectional dimension of the energy delivery portion 27 is configured for sliding receipt in the ablation lumen 25 of the ablation sheath 22 in a manner positioning the directional ablative energy, emitted by the energy delivery portion, continuously toward the predetermined contact surface 23 of the ablation sheath 22. In one example, as shown in FIGURES 8 and 9, the transverse peripheral dimensions of the energy delivery portion 27 and the ablation lumen 25 are generally D-shaped, and substantially similar in dimension. Thus, the window portion 58 of the insulator 61, as will be discussed, is preferably semi-cylindrical and concentric with the interior wall 62 defining the ablation lumen 25 of the ablation sheath 22. It will be appreciated, however, that any geometric configuration may be applied to ensure unitary or aligned insertion. As another example, one of the energy delivery portion and the interior wall of the ablation lumen may include a key member and corresponding receiving groove, or the like. Such key and receiving groove designs, nonetheless, should avoid relatively sharp edges to enable smooth advancement and retraction of the energy delivery portion in the ablation lumen 25.

This dimension alignment relationship can be maintain along the length of the predetermined contact surface of the ablation sheath 22 as the energy delivery portion 27 is advanced through the ablation lumen whether in the configuration of FIGURES 2, 6, 7 or 12. In this manner, a physician may determine that once the predetermined contact surface 23 of the ablation sheath 22 is properly oriented and positioned adjacent or in contact against the targeted tissue 21, the directional component (as will be discussed) of the energy delivery portion 27 will then be automatically aligned with the targeted tissue as it is advanced through the ablation lumen 25. Upon selected ablation by the ablative energy, a series of overlapping lesions 44-44"' (FIGURES 13A-13D) or a single continuous lesion can then be generated.

It will further be appreciated that the dimensional tolerances therebetween should be sufficiently large to enable smooth relative Advancement and retraction of the energy delivery portion 27 around curvilinear geometries, and further enable the passage of gas therebetween. Since the ablation lumen 25 of the ablation sheath 22 is closed ended, gases must be permitted to flow between the energy delivery portion 27 and the interior wall 62 defining the ablation lumen 25 to avoid the compression of gas during advancement of the energy delivery portion therethrough. Moreover, the tolerance must be sufficiently small to substantially prevent axial rotation of the energy delivery portion in the ablation lumen 25 for alignment purposes. The dimensional tolerance between the transverse cross-sectional periphery of the ablation lumen and that of the substantially conforming energy delivery portion 27, for instance, should be in the region of about 0.02 mm (0.001 inches) to about 0.13 mm (0.005 inches).

To further facilitate preservation of the fluids and tissues along the backside of the ablation sheath 22 (i.e., the side opposite the contact surface 23 of the sheath), a thermal isolation component (not shown) is disposed longitudinally along, and substantially adjacent to, the ablation lumen 25. Thus, during activation of the ablative device, the isolation component and the directive component 73 of the energy ablation portion 27 cooperate to form a thermal barrier along the backside of the ablation sheath.

For instance, the isolation component may be provided by an air filled isolation lumen extending longitudinally along, and substantially adjacent to, the ablation lumen 25. The cross-sectional dimension of the isolation lumen may be C-shaped or crescent shaped to partially surround the ablation lumen 25. In another embodiment, the isolation lumen may be filled with a thermally refractory material.

In still another embodiment, a circulating fluid, which is preferably biocompatible, may be disposed in the isolation lumen to provide to increase the thermal isolation. Two or more lumens may be provided to increase fluid flow. One such biocompatible fluid providing suitable thermal properties is saline solution.

Similar to the composition of the guide sheath 52, the ablation sheath 22 is composed of a flexible bio-compatible material, such as PU Pellethane, Teflon or polyethylene, which is capable of shape retention once external forces acting on the sheath are removed. By way of example, when the distal portions of the ablation sheath 22 are advanced past the interior walls of the guide lumen 54 of the guide sheath 52, the ablation sheath 22 will return to its preformed shape in the interior of the Heart.

To facilitate shape retention, the ablation sheath 22 preferably includes a shape retaining member 63 extending longitudinally through the distal portions of the ablation sheath where shape retention is necessary. As illustrated in FIGURES 1, 8 and 9, this retaining member 63 is generally extends substantially parallel and adjacent to the ablation lumen 25 to reshape the predetermined contact surface 23 to its desired pre-shaped form once the restraining forces are removed from the sheath. While this shape-memory material must be sufficiently resilient for shape retention, it must also be sufficiently bendable to enable insertion through the guide lumen 54 of the guide sheath 52. In the preferred form, the shape retaining member is composed of a superelastic metal, such as Nitinol (NiTi). Moreover, the preferred diameter of this material should be in the range of 0.020 inches to about 0.050 inches, and more preferably about 0.035 inches.

When used during a surgical procedure, the ablation sheath 22 is preferably transparent which enables a surgeon to visualize the position of the energy delivery portion 27 of the ablative device 26 through an endoscope or the like. Moreover, the material of ablation sheath 22 must be substantially unaffected by the ablative energy emitted by the energy delivery portion 27. Thus, as will be apparent, depending upon the type of energy delivery portion and the ablative source applied, the material of the tubular sheath must exhibit selected properties, such as a low loss tangent, low water absorption or low scattering coefficient to name a few, to be unaffected by the ablative energy.

As previously indicated, the ablation sheath 22 is advanced and oriented, relative to the guide sheath 52, adjacent to or into contact with the targeted tissue region 21 to form a series of overlapping lesions 44-44"', such as those illustrated in FIGURES 3 and 13A-13D. Preferably, the contact surface 23 of the pre-shaped ablation sheath 22 is negotiated into physical contact with the targeted tissue 21. Such contact increases the precision of the tissue ablation while further facilitating energy transfer between the ablation element and the tissue to be ablated, as will be discussed.

To assess proper contact and positioning of the contact surface 23 of the ablation sheath 22 against the targeted tissue 21, at least one positioning electrode, generally designated 64, is disposed on The exterior surface of the ablation sheath for contact with the tissue. Preferably a plurality of electrodes arc positioned along and adjacent the contact surface 23 to assess contact of the elongated and three dimensionally shaped contact surface. These electrodes 64 essentially measure whether there is any electrical activity (or electrophysiological signals) to one or the other side of the ablation sheath 22. When a strong electrical activation signal is detected, or inter-electrode impedance is measured when two or more electrodes are applied, contact with the tissue can be assessed. Once the physician has properly situated and oriented the sheath, they may commence advancement of the energy delivery portion 27 through the ablation lumen 25. Additionally, these positioning electrodes may be applied to map the biological tissue prior to or after an ablation procedure, as well as be used to monitor the patient's condition during the ablation process.

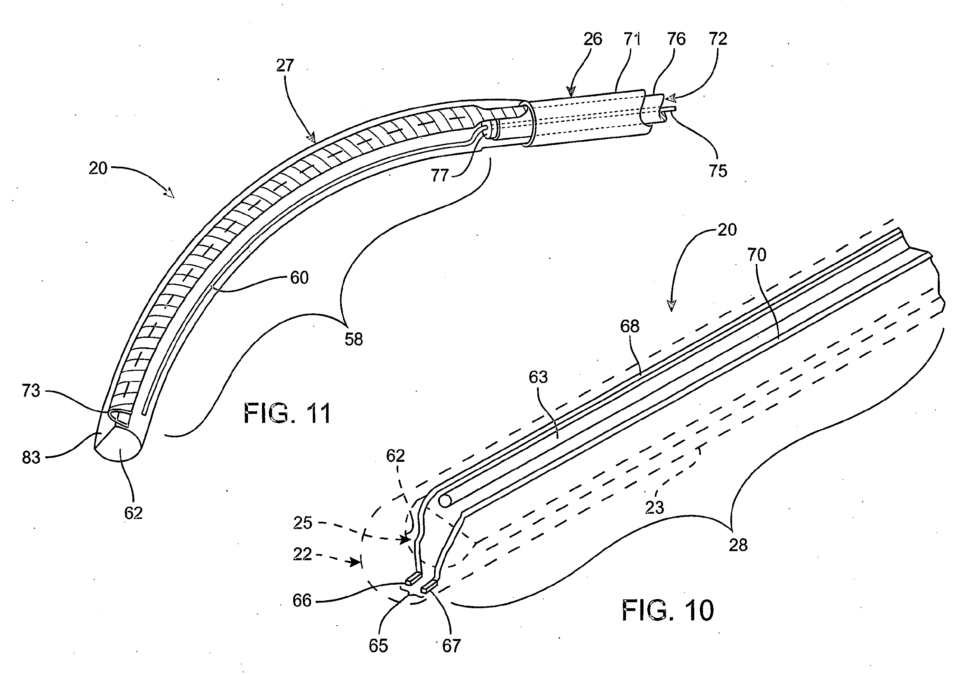

To facilitate discussion of the above aspects of the present invention, FIGURE 10 illustrates two side-by-side electrodes configured for sensing electrical activity in substantially one direction, in accordance with one aspect of the present invention. This electrode arrangement generally includes a pair of longitudinally extending electrode elements 66, 67 that are disposed on the outer periphery of the ablation sheath 22. The pair of electrode elements 66, 67 are positioned side by side and arranged to be substantially parallel to one another. In general, splitting the electrode arrangement into a pair of distinct elements permits substantial improvements in the resolution of the detected electrophysiological signals. Therefore, the pair of electrode elements 66, 67 are preferably spaced apart and electrically isolated from one another. It will be appreciated, however, that only one electrode may be employed to sense proper tissue contact. It will also be appreciated that ring or coiled electrodes can also be used.

The pair of electrode elements 66, 67 are further arranged to be substantially parallel to the longitudinal axis of the ablation sheath 22. In order to ensure that the electrode elements are sensing electrical activity in substantially the same direction, the space between electrodes should be sufficiently small. It is generally believed that too large space may create problems in determining the directional position of the catheter and too small a space may degrade the resolution of the detected electrophysiological signals. By way of example, the distance between the two pair of electrode elements may be between about 0.5 and 2.0 mm.

The electrode elements 66, 67 are preferably positioned substantially proximate to the predetermined contact surface 23 of the ablation sheath 22. More preferably, the electrode elements 66, 67 are positioned just distal to the distal end of the predetermined contact surface 23 since it is believed to be particularly useful to facilitate mapping and monitoring as well as to position the ablation sheath 22 in the area designated for tissue ablation. For example, during some procedures, a surgeon may need to ascertain where the distal end of the ablation sheath 22 is located in order to ablate the appropriate tissues. In another embodiment, the electrode elements 66, 67 may be positioned substantially proximate the proximal end of the predetermined contact surface 23, at a central portion of the contact surface 23 or a combination thereof. For instance, when attempting to contact the loop-shaped ablation sheath 22 employed to isolate each of left and inferior pulmonary vein orifices 37, 38, a central location of the electrodes along the looped-shape contact surface 23 may best sense contact with the targeted tissue. Moreover, while not specifically illustrated, a plurality of electrode arrangements may be disposed along the ablation sheath as well. By way of example, a first set of electrode elements may be disposed distally from the predetermined contact surface, a second set of electrode elements may be disposed proximally to the contact surface, while a third set of electrode elements may be disposed centrally thereof. These electrodes may also be used with other types of mapping electrodes, for example, a variety of suitable mapping electrode arrangements are described in detail in U.S. Patent No. 5,788,692 to Campbell, et al. Although only a few positions have been described, it should be understood that the electrode elements may be positioned in any suitable position along the length of the ablation sheath.

The electrode elements 66, 67 may be formed from any suitable material, such as stainless steel and iridium platinum. The width (or diameter) and the length of the electrode may vary to some extent based on the particular application of the catheter and the type of material chosen. Futhcrmore, in the preferred embodiment where microwave is used as the ablative energy, the electrodes are preferably dimensioned to minimize electromagnetic filed interference, for example, the capturing of the microwave field produced by the antenna. In most embodiments, the electrodes are arranged to have a length that is substantially larger than the width, and are preferably between about 0.25 mm (0.010 inches) to about 0.63 mm (0.025 inches) and a length between about 1.3 mm (0.50 inch) to about 2.5 mm (1.0 inch).

Although the electrode arrangement has been shown and described as being parallel plates that are substantially parallel to the longitudinal axis of the ablation sheath 22 and aligned longitudinally (e.g., distal and proximal ends match up), it should be noted that this is not a limitation and that the electrodes can be configured to be angled relative to the longitudinal axis of the ablation sheath 22 (or one another) or offset longitudinally. Furthermore, although the electrodes have been shown and described as a plate, it should be noted that the electrodes may be configured to be a wire or a point such as a solder blob.

Each of the electrode elements 66, 67 is electrically coupled to an associated electrode wire 68, 70 and which extend through ablation sheath 22 to at least the proximal portion of the flexible outer tubing. In most embodiments, the electrode wires 68, 70 are electrically isolated from one another to prevent degradation of the electrical signal, and arc positioned on opposite sides of the retaining member 63. The connection between the electrodes 64, 65 and the electrode wires 68, 70 may be made in any suitable manner such as soldering, brazing, ultrasonic welding or adhesive bonding. In other embodiments, the longitudinal electrodes can be formed from the electrode wire itself. Forming the longitudinal electrodes from the electrode wire, or out of wire in general, is particularly advantageous because the size of wire is generally small and therefore the longitudinal electrodes elements may be positioned closer together thereby forming a smaller arrangement that takes up less space. As a result, the electrodes may be positioned almost anywhere on a catheter or surgical tool. These associated electrodes are described in greater detail in U.S. Patent No 6,673,068.

Referring now to FIGURES 1, 8, 9 and 11, the ablative device 26 is preferably in the form of an elongated member, which is designed for insertion into the ablation lumen 25 of the ablation sheath 22, and which in turn is designed for insertion into a vessel (such as a blood vessel) in the body of a patient. It will be understood, however, that the present invention may be in the form of a handheld instrument for use in open surgical or minimally invasive procedures (FIGURES 12).

The ablative device 26 typically includes a flexible outer tubing 71 (having one or several lumens therein), a transmission line 72 that extends through the flexible tubing 71 and an energy delivery portion 27 coupled to the distal end of the transmission line 72. The flexible outer tubing 71 may be made of any suitable materials such as medical grade polyolefins, fluoropolymers, or polyvinylidene fluoride. By way of example, PEBAX resins from Autochem of Germany have been used with success for the outer tubing of the body of the catheter.

In accordance with another aspect of the present invention, the ablative energy emitted by the energy delivery portion 27 of the ablative device 26 may be one of several types. Preferably, the energy delivery portion 27 includes a microwave component which generates a electromagnetic field sufficient to cause tissue ablation. As mentioned, as will be discussed in greater detail below, the ablative energy may also be derived from a laser source, a cryogenic source, an ultrasonic source or a radiofrequency source, to name a few.

Regardless of the source of the energy, a directive component cooperates with the energy source to control the direction and emission of the ablative energy. This assures that the surrounding tissues of the targeted tissue regions will be preserved. Further, the use of a directional field has several potential advantages over conventional energy delivery structure that generate uniform fields about the longitudinal axis of the energy delivery portion. For example, in the microwave application, by forming a more concentrated and directional electromagnetic field, deeper penetration of biological tissues is enabled, and the targeted tissue region may be ablated without heating as much of the surrounding tissues and/or blood. Additionally, since substantial portions the radiated ablative energy is not emitted in the air or absorbed in the blood or the surrounding tissues , less power is generally required from the power source, and less power is generally lost in the microwave transmission line.

In the preferred form, the energy delivery portion 27 of the ablative device 26 is an antenna assembly configured to directionally emit a majority of an electromagnetic field from one side thereof. The antenna assembly 27, as shown in FIGURES 9 and 11, preferably includes a flexible antenna 60, for generating the electromagnetic field, and a flexible reflector 73 as a directive component, for redirecting a portion of the electromagnetic field to one side of the antenna opposite the reflector. Correspondingly, the resultant electromagnetic field includes components of the originally generated field, and components of the redirected electromagnetic field. During aligned insertion of the antenna assembly 27 into the ablation lumen 25, via the key structure 48, the directional field will thus be continuously aligned toward the contact surface 23 of the ablation sheath 22 as the antenna assembly is incrementally advanced through the ablation lumen 25.

FIGURE 11 illustrates that the proximal end of the antenna 60 is preferably coupled directly or indirectly to the inner conductor 75 of a coaxial transmission line 72. A direct connection between the antenna 60 and the inner conductor 75 may be made in any suitable manner such as soldering, brazing, ultrasonic welding or adhesive bonding. In other embodiments, antenna 60 can be formed from the inner conductor 75 of the transmission line 72 itself. This is typically more difficult from a manufacturing standpoint but has the advantage of forming a more rugged connection between the antenna and the inner conductor. As will be described in more detail below, in some implementations, it may be desirable to indirectly couple the antenna to the inner conductor through a passive component, such a capacitor, an inductor or a stub tuner for example, in order to provide better impedance matching between the antenna assembly and the transmission line, which is a coaxial cable in the preferred embodiment.

Briefly, the transmission line 72 is arranged for actuating and/or powering the antenna 60. Typically, in microwave devices, a coaxial transmission line is used, and therefore, the transmission line 72 includes an inner conductor 75, an outer conductor 76, and a dielectric material 77 disposed between the inner and outer conductors. In most instances, the inner conductor 75 is coupled to the antenna 60. Further, the antenna 60 and the reflector 73 are enclosed (e.g., encapsulated) in a flexible insulative material thereby forming the insulator 61, to be described in greater detail below, of the antenna assembly 27.

The power supply (not shown) includes a microwave generator which may take any conventional form. When using microwave energy for tissue ablation, the optimal frequencies are generally in the neighborhood of the optimal frequency for heating water. By way of example, frequencies in the range of approximately 800 MHz to 6 GHz work well. Currently, the frequencies that are approved by the Federal Communication Commission (FCC) for experimental clinical work includes 915 MHz and 2.45 GHz. Therefore, a power supply having the capacity to generate microwave energy at frequencies in the neighborhood of 2.45 GHz may be chosen. A conventional magnetron of the type commonly used in microwave ovens is utilized as the generator. It should be appreciated, however, that any other suitable microwave power source could be substituted in its place, and that the explained concepts may be applied at other frequencies like about 434 MHz or 5.8 GHz (ISM band).

In the preferred embodiment, the antenna assembly 27 includes a longitudinally extending antenna wire 60 that is laterally offset from the transmission line inner conductor 75 to position the antenna closer to the window portion 58 of the insulator 61 upon which the directed electric field is transmitted. The antenna 60 illustrated is preferably a longitudinally extending exposed wire that extends distally (albeit laterally offset) from the inner conductor. However it should be appreciated that a wide variety of other antenna geometries may be used as well. By way of example, helical coils, flat printed circuit antennas and other antenna geometries will work as well.

Briefly, the insulator 61 is preferably provided by a good, low-loss dielectric material which is relatively unaffected by microwave exposure, and thus capable of transmission of the electromagnetic field therethrough. Moreover, the insulator material preferably has a low water absorption so that it is not itself heated by the microwaves. Incidentally, when the emitted ablative energy is microwave in origin, the ablation sheath must also include these material properties. Finally, the insulation material must be capable of substantial flexibility without fracturing or breaking. Such materials include moldable TEFLON^{®} , silicone, or polyethylene, polyimide, etc.

As will be appreciated by those familiar with antenna design, the field generated by the illustrated antenna will be generally consistent with the length of the antenna. That is, the length of the electromagnetic field is generally constrained to the longitudinal length of the antenna. Therefore, the length of the field may be adjusted by adjusting the length of the antenna. Accordingly, microwave ablation elements having specified ablation characteristics can be fabricated by building them with different length antennas. Additionally, it should be understood that longitudinally extending antennas are not a requirement and that other shapes and configurations may be used.

The antenna 60 is preferably formed from a conductive material. By way of example, copper or silver-plated metal work well. Further, the diameter of the antenna 60 may vary to some extent based on the particular application of the catheter and the type of material chosen. In microwave systems using a simple exposed wire type antenna, for instance, wire diameters between about 0.25 mm (0.010) to about 0.5 mm (0.020 inches) work well. In the illustrated embodiment, the diameter of the antenna is about 0.3 mm (0.013 inches).

In a preferred embodiment, the antenna 60 is positioned closer to the area designated for tissue ablation in order to achieve effective energy transmission between the antenna 60 and the targeted tissue 21 through the predetermined contact surface 23 of the ablation sheath 22. This is best achieved by placing the antenna 60 proximate to the outer peripheral surface of the antenna insulator 61. More specifically, a longitudinal axis of the antenna 60 is preferably off-set from, but parallel to, a longitudinal axis 78 of the inner conductor 75 in a direction away from the reflector 73 and therefore towards the concentrated electromagnetic field (FIGURES 8 and 9). By way of example, placing the antenna between about 0.25 mm (0.010 inches) to about 0.5 mm (0.020 inches) away from the outer peripheral surface of the antenna insulator works well. In the illustrated embodiment, the antenna is about 0.3 mm (0.013 inches) away from the outer peripheral surface of the antenna insulator 61. However, it should be noted that this is not a requirement and that the antenna position may vary according to the specific design of each catheter.

Referring now to the directive component or reflector 73, it is positioned adjacent and generally parallel to a first side of the antenna, and is configured to redirect those components of the electromagnetic field contacting the reflector back towards and out of a second side of the antenna assembly 27 opposite the reflector. A majority of the electromagnetic field, consequently, is directed out of the window portion 58 of the insulator 61 in a controlled manner during ablation.

To reduce undesirable electromagnetic coupling between the antenna and the reflector 73, the antenna 60 is preferably off-set from the reflector 73 (FIGURES 8 and 9). This off-set from the longitudinal axis 78 further positions the antenna 60 closer to the window portion 58 to facilitate ablation by positioning the antenna 60 closer to the targeted tissue region. It has been found that the minimum distance between the reflector and the antenna may be between about 0.020 to about 0.030 inches, in the described embodiment, in order to reduce the coupling. However, the distance may vary according to the specific design of each ablative device.

The proximal end of the reflector 73 is preferably coupled to the outer conductor 76 of the coaxial transmission line 72. Connecting the reflector to the outer conductor serves to better define the electromagnetic field generated during use. That is, the radiated field is better confined along the antenna, to one side, when the reflector is electrically connected to the outer conductor of the coaxial transmission line. The connection between the reflector 73 and the outer conductor 76 may be made in any suitable manner such as soldering, brazing, ultrasonic welding or adhesive bonding. In other embodiments, the reflector can be formed from the outer conductor of the transmission line itself. This is typically more difficult from a manufacturing standpoint but has the advantage of forming a more rugged connection between the reflector and the outer conductor.

In one embodiment, to improve flexibility at the electrical connection with the outer conductor 76 and entirely along the energy delivery device, the proximal end of the reflector 73 is directly contacted against the outer conductor without applying solder or such conductive adhesive bonding. In this design, the insulator material of the insulator 61 functions as the adhesive to maintain electrical continuity. This is performed by initially molding the antenna wire in the silicone insulator. The reflector 73 is subsequently disposed on the molded silicone tube, and is extended over the outer conductor 76 of coaxial cable transmission line 72. A heat shrink tube is then applied over the assembly to firmly maintain the electrical contact between the reflector 73 and the coaxial cable outer conductor 76. In other embodiments, the reflector may be directly coupled to a ground source or be electrically floating.

As previously noted, the antenna 60 typically emits an electromagnetic field that is fairly well constrained to the length of the antenna. Therefore, in some embodiments, the distal end of the reflector 73 extends longitudinally to at about the distal end of the antenna 60 so that the reflector can effectively cooperate with the antenna. This arrangement serves to provide better control of the electromagnetic field during ablation. However, it should be noted that the actual length of the reflector may vary according to the specific design of each catheter. For example, catheters having specified ablation characteristics can be fabricated by building catheters with different length reflectors.

Furthermore, the reflector 73 is typically composed of a conductive, metallic material or foil. However, since the antenna assembly 27 must be relatively flexible in order to negotiate the curvilinear ablation lumen 25 of the ablation sheath 22 as the ablative device it is advanced therethrough, the insulator 61, the antenna wire and the reflector must collectively be relatively flexible. Thus, one particularly material suitable for such a reflector is a braided conductive mesh having a proximal end conductively mounted to the distal portion of the outer conductor of the coaxial cable. This conductive mesh is preferably thin walled to the shield assembly yet provide the appropriate microwave shielding properties, as well as enable substantial flexibility of the shield device during bending movement. For example, a suitable copper mesh wire should have a diameter in the range of about 0.005 inches to about 0.010 inches, and more preferably about 0.007 inches. A good electrical conductor is generally used for the shield assembly in order to reduce the self-heating caused by resistive losses. Such conductors includes, but are not restricted to copper, silver and gold.

Another suitable arrangement may be thin metallic foil reflector 73 which is inherently flexible. However, to further increase flexibility, the foil material can be pleated or folded which resists tearing during bending of the antenna assembly 27. These foils can be composed of copper that has a layer of silver plating formed on its inner peripheral surface. Such silver plating, which can also be applied to the metallic mesh material, is used to increase the conductivity of the reflector. It should be understood, however, that these materials are not a limitation. Furthermore, the actual thickness of the reflector may vary according to the specific material chosen.

Referring back to FIGURE 11, the reflector 73 is preferably configured to have an arcuate or meniscus shape (e.g., crescent), with an arc angle that opens towards the antenna 60. Flaring the reflector towards the antenna serves to better define the electromagnetic field generated during use. Additionally, the reflector functions to isolate the antenna 60 from the restraining member 63 of the ablation sheath 22 during ablation. Since the restraining member 63 is preferably metallic in composition (most preferably Nitinol), it is desirable minimize electromagnetic coupling with the antenna. Thus, the reflector 73 is preferably configured to permit at most a 180° circumferential radiation pattern from the antenna. In fact, it has been discovered that arc angles greater than about 180° are considerably less efficient. More preferably, the arc angle of the radiation pattern is in the range of about 90° to about 120°.

While the reflector is shown and described as having an arcuate shape, it will be appreciated that a plurality of forms may be provided to accommodate different antenna shapes or to conform to other external factors necessary to complete a surgical procedure. For example, any flared shape that opens towards the antenna may work well, regardless of whether it is curvilinear or rectilinear.

Further still, it should be noted that the shape of the reflector need not be uniform. For example, a first portion of the reflector (e.g., distal) may be configured with a first shape (e.g., 90° arc angle) and a second portion (e.g., proximal) of the reflector may be configured with a second shape (e.g., 120° arc angle). Varying the shape of the reflector in this manner may be desirable to obtain a more uniform radiated field. It is believed that the energy transfer between the antenna and the tissue to be ablated tends to increase by decreasing the coverage angle of the reflector, and conversely, the energy transfer between the antenna and the tissue to be ablated tends to decrease by increasing the coverage angle of the reflector. Accordingly, the shape of the reflector may be altered to balance out non-uniformities found in the radiated field of the antenna arrangement.

In another configuration, the directive component 73 for the microwave antenna assembly 27 can be provided by another dielectric material having a dielectric constant different than that of the insulator material 67. Indeed, a strong reflection of electromagnetic wave is observed when the wave reaches an interface created by two materials with a different dielectric constant. For example, a ceramic loaded polymer can have a dielectric constant comprised between 15 and 55, while the dielectric of a fluoropolymer like Teflon or is comprised between 2 and 3. Such an interface would create a strong reflection of the wave and act as a semi-reflector.

It should also be noted that the longitudinal length of the reflector need not be uniform. That is, a portion of the reflector may be stepped towards the antenna or a portion of the reflector may be stepped away from the antenna. Stepping the reflector in this manner may be desirable to obtain a more uniform radiated field. While not wishing to be bound by theory, it is believed that by placing the reflector closer to the antenna, a weaker radiated field may be obtained, and that by placing the reflector further away from the antenna, a stronger radiated field may be obtained. Accordingly, the longitudinal length of the reflector may be altered to balance out non uniformities found in the radiated field of the antenna arrangement. These associated reflectors are described in greater detail in U.S. Patent No 6,245,062.

In a typical microwave ablation system, it is important to match the impedance of the antenna with the impedance of the transmission line. As is well known to those skilled in the art, if the impedance is not matched, the catheter's performance tends to be well below the optimal performance. The decline in performance is most easily seen in an increase in the reflected power from the antenna toward the generator. Therefore, the components of a microwave transmission system are typically designed to provide a matched impedance. By way of example, a typical set impedance of the microwave ablation system may be on the order of fifty (50) ohms.

Referring back to FIGURES 10 and 11, and in accordance with one embodiment of the present invention, an impedance matching device may be provided to facilitate impedance matching between the antenna 60 and the transmission line 72. The impedance matching device is generally disposed - proximate the junction between the antenna 60 and the inner conductor 75. For the most part, the impedance match is designed and calculated assuming that the antenna assembly 27, in combination with the predetermined contact surface 23 of the ablation sheath 22, is in resonance to minimize the reflected power, and thus increase the radiation efficiency of the antenna structure.

In one embodiment, the impedance matching device is determined by using a Smith Abacus Model. In the Smith Abacus Model, the impedance matching device may be ascertained by measuring the impedance of the antenna with a network analyzer, analyzing the Measured value with a Smith Abacus Chart, and selecting the appropriate device. By way of example, the impedance matching device may be any combination of a capacitor, resistor, inductor, stub tuner or stub transmission line, whether in series or in parallel with the antenna. An example of the Smith Abacus Model is described in Reference: David K. Cheng, "Field and Wave Electromagnetics," second edition, Addison-Wesley Publishing, 1989. In one preferred implementation, the impedance matching device is a serial capacitor having a capacitance in the range of about 0.6 to about 1.0 picoFarads. In the illustration shown, the serial capacitor has a capacitance of about 0.8 picoFarads.

As above-mentioned, the impedance will be matched assuming flush contact between the antenna assembly 27 and the ablation sheath (FIGURE 9). As the antenna assembly 27 is advanced through the ablation lumen 25, before selective ablation, it is desirable to position the window portion 58 of the flexible antenna insulator 61 in flush contact against the interior wall 62 of the ablation lumen 25, opposite the predetermined contact surface 23. This arrangement may substantially reduce the impedance variance caused by the interface between insulator 61 and the ablation sheath 22 as the directional field is transmitted therethrough. In comparison, if the window portion 58 were not required to be positioned in flush contact against the interior wall 62 of the ablation lumen, pockets of air or fluid, or the like, may be disposed intermittently therebetween which would result in a greater degree of impedance variations at this interface. Consequently, the above-indicated impedance matching techniques would be less effective.

To assure such flush contact during selective directional ablation and advancement along the sheath ablation lumen, the ablation system 20 preferably incorporates a forcing mechanism 81 (FIGURES 8 and 9) adapted to urge the window portion 58 of the antenna assembly 27 into flush contact against the interior wall 62 of the ablation sheath. Preferably, the forcing mechanism cooperates between a support portion 82 of the interior wall 62 of the ablation lumen 25 and the forcing wall portion 83 of the antenna assembly.

When not operational, the forcing mechanism permits relative axial displacement between the ablative device 26 and the ablation sheath for repositioning of the antenna assembly 27 along the ablation path 28 (FIGURE 8). Upon selective operation, the forcing mechanism 81 contacts the forcing wall portion 83 to urge window portion 58 flush against the interior wall 62 opposite the predetermined contact surface 23. Consequently, the impedance match between the antenna and the transmission line is properly achieved and stable even when the antenna is moving in the ablation sheath.

In one embodiment, the forcing mechanism may be provided by an inflatable structure acting between the support portion 82 of the interior wall 62 of the ablation lumen 25 and the forcing wall portion 83 of the antenna assembly device. Upon selective inflation of forcing mechanism 81 (FIGURE 9), the window portion 58 will be urged into flush contact with the interior wall 62 of the ablation lumen. Upon selective deletion of the forcing mechanism 81 (FIGURE 8), relative axial displacement between the antenna assembly 27 and the ablation sheath may commence. The forcing mechanism can be provided by other techniques such as spring devices or the like.

In accordance with another aspect of the present invention, the ablative energy may be in the form of laser energy sufficient to ablate tissue. Example of such laser components include CO₂ or Nd: YAG lasers. To transmit the beams, the transmission line 72 is preferably in the form of a fiber optic cable or the like.

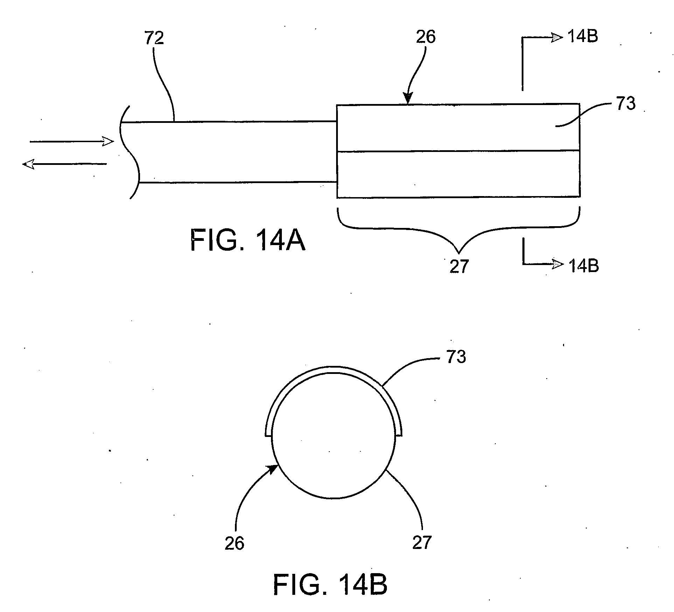

In this design, as shown in FIGURES 14A and 14B, the directive component 73 may be provided by a reflector having a well polished smooth reflective or semi-reflective surface. This preferably metallic reflective surface is configured to reflect the emitted laser energy toward the targeted tissue region. By way of example, functional metallic materials include silver or platinum. In another configuration, similar to the difference in dielectric constants of the microwave ablation device 26, the directive component of the laser ablative device may be provided between two layers of dielectric materials with a sufficient difference between the refractory indexes. Here, at least one dielectrics directive component layer functions like the outer dielectric layer of the fiber optic transmission line 72 to obtain "total internal reflection". Consequently, the laser energy can be emitted away from the dielectric layer. By providing more than one dielectric layer, "total internal reflection" may be attained at several angles of incidence. Again, the reflection of the electromagnetic wave is caused by the interface between two media having different dielectric constants. Generally speaking, the higher is the difference between the dielectric constants, the more significant is the internal reflection. In addition, when more than one dielectric layer are involved, interference can be used to direct the laser energy in a preferred direction.

Moreover, when the ablative energy is laser based, it will be appreciated that it is desirable that both the ablation sheath 22 and the ablation device be composed of materials which have a low scattering coefficient and a low factor of absorption. In addition, it is also preferable to use material with low water absorption.

It will be appreciated that a plurality of designs can be used for the laser energy delivery portion. For example, the laser energy delivery portion can consist of multiple reflective particles embedded in a laser transparent material. The laser wave is propagating from the laser generator to the optic fiber transmission line and enter in the laser energy delivery portion. The embedded reflective particles diffracts the light, which is reflected toward the tissue to be ablated by the directive component 73.

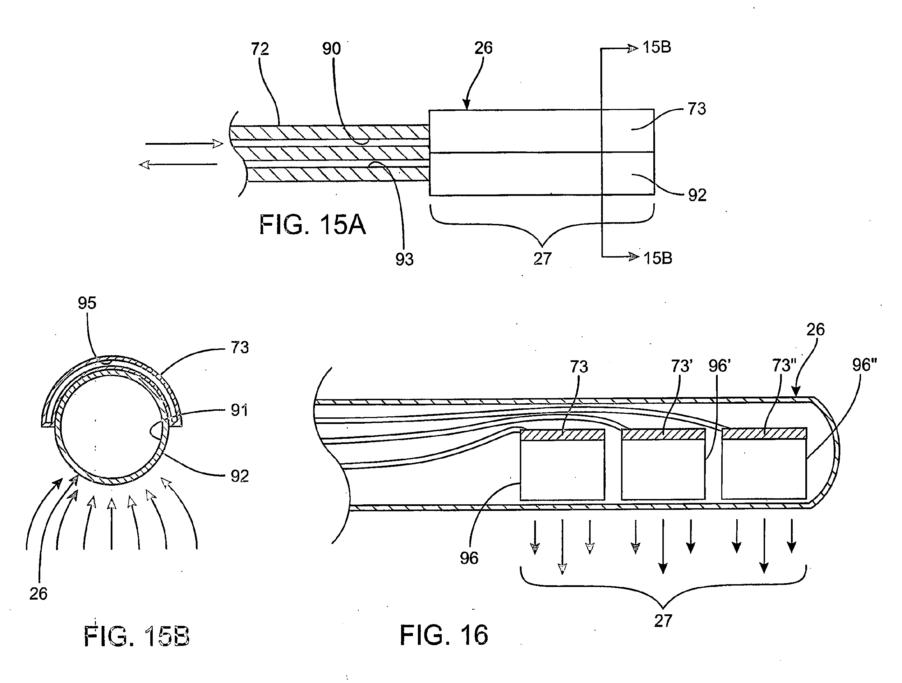

In yet another alternative embodiment, cryogenic energy may be employed as an ablative energy. Briefly, as shown in FIGURES 15A and 15B, in these cryogenic ablation device designs, a cryogenic fluid, such as a pressurized gas (E.g., Freon) is passed through an inflow lumen 90 in the ablation device transmission line 72. The distal ablative device 26 is preferably provided by a decompression chamber which decompresses the pressurized gas from the inflow lumen 90 therein. Upon decompression or expansion of the pressurized gas in the decompression chamber 91, the temperature of the exterior surface 92 of the decompression chamber is sufficiently reduced to cause tissue ablation upon contact thereof. The decompressed gas is then exhausted through the outflow lumen 93 of the transmission line 72.

FIGURE 15B illustrates that the directive component 73 is in the form of a thermal insulation layer extending longitudinally along one side of the energy delivery portion 27. By forming a good thermal insulator with a low thermal conductivity, the C-shaped insulation layer 73 will substantially minimize undesirable cryogenic ablation of the immediate tissue surrounding of the targeted tissue region. In one configuration, the isolation layer may define a thin, elongated gap 95 which partially surrounds the decompression chamber 91. This gap 95 may then be filled with air, or an inert gas, such as CO₂, to facilitate thermal isolation. The isolation gap 95 may also be filled with a powder material having relatively small solid particulates or by air expended polymer. These materials would allow small air gaps between the insulative particles or polymeric matrix for additional insulation thereof. The isolation layer may also be provided by a refractory material. Such materials forming an insulative barrier include ceramics, oxides, etc.

Referring now to FIGURE 16, an ultrasound ablation device may also be applied as another viable source of ablation energy. For example, a piezoelectric transducer 96 may be supplied as the ablative element which delivers acoustic waves sufficient to ablate tissue. These devices emit ablative energy which can be directed and shaped by applying a directive echogenic component to reflect the acoustic energy. Moreover, a series or array of piezoelectric transducers 96, 96' and 96" can be applied to collectively form a desired radiation pattern for tissue ablation. For example, by adjusting the delay between the electrical exciting signal of one transducer and its neighbor, the direction of transmission can be modified. Typical of these transducers include piezoelectric materials like quartz, barium oxides, etc.

In this configuration, the directive component 73 of the ultrasonic ablation device may be provided by an echogenic material (73-73") positioned proximate the piezoelectric transducers. This material reflects the acoustic wave and which cooperates with the transducers to direct the ablative energy toward the targeted tissue region. By way of example, such echogenic materials are habitually hard. They include, but are not restricted to metals and ceramics for example.

Moreover, when the ablative energy is ultrasonic based, it will be appreciated that it is desirable that both the ablation sheath 22 and the ablation device be composed of materials which have low absorption of the acoustic waves, and that provide a good acoustic impedance matching between the tissue and the transducer. In that way, the thickness and the material chosen for the ablation sheath play in important role to match the acoustic properties of the tissue to be ablated and the transducer. An impedance matching jelly can also be used in the ablation sheath to improve the acoustic impedance matching.

Lastly, the ablation device may be provided by a radiofrequency (RF) ablation source which apply RF conduction current sufficient to ablate tissue. These conventional ablation instruments generally apply conduction current in the range of about 450 kHz to about 550 kHz. Typical of these RF ablation devices include ring electrodes, coiled electrodes or saline electrodes.

To selectively direct the RF energy, the directive component is preferably composed of an electrically insulative and flexible material, such as plastic or silicone. These biocompatible materials perform the function of directing the conduction current toward a predetermined direction.

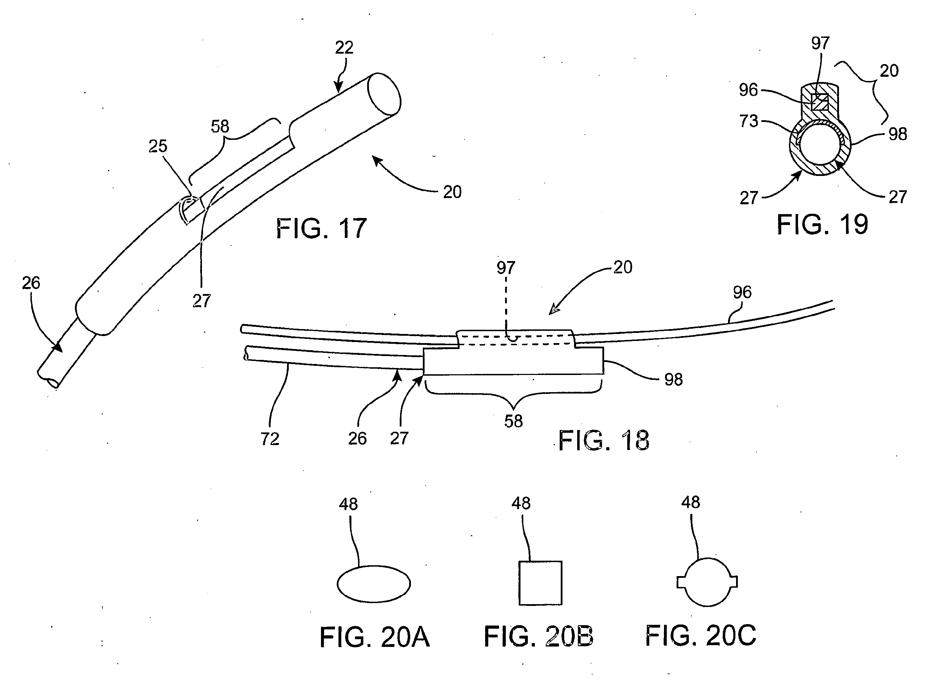

In an alternative embodiment, as best viewed in FIGURE 17, the window portion 58 of the ablation sheath 22 is provided by an opening in the sheath along the ablation path, as opposed to being merely transparent to the energy ablation devices. In this manner, when the ablation sheath 22 is properly positioned with the window portion placed proximate and adjacent the targeted tissue, the energy delivery portion 27 of the ablation device 26 may be slideably positioned into direct contact with the tissue for ablation thereof. Such direct contact is especially beneficial when it is technically difficult to find a sheath that is merely transparent to the used ablative energy. For example, it would be easier to use a window portion when RF energy is used. The ablative RF element could directly touch the tissue to be ablated while the directive element would be the part of the ablation sheath 22 facing away the window portion 58. Furthermore, during surgical ablation, the window portion could be used by the surgeon to indicate the area where an ablation can potentially be done with the energy ablation device.

In yet another embodiment, the ablation system 20 may be in the form of a rail system including a rail device 96 upon which the ablation device 26 slides therealong as compared to therethrough. FIGURES 18 and 19 illustrate the rail device 96 which is preferably pre-shaped or bendable to proximately conform to the surface of the targeted tissue. Once the rail device 96 is positioned, the ablation device can be advanced or retracted along the path defined by the rail device for ablation of the targeted tissue 21.

The ablation device 26 in this arrangement includes a body portion 98 housing the energy delivery portion 27 therein. The window portion 58 is preferably extend longitudinally along the outer surface of one side of the housing. An opposite side of the housing, and longitudinally oriented substantially parallel to the window portion 58 is a rail receiving passage 97 formed and dimensioned to slideably receive and slide over the rail device 96 longitudinally therethrough. In one configuration, the energy delivery portion 27 may be advanced by pushing the body portion 98 through the transmission line 72. Alternatively, the energy delivery portion 27 may be advanced by pulling the body portion 98 along the path of the rail system 20.

As best viewed in FIGURE 19, the directive component. 73 of the ablation device 26 is integrally formed with the body portion 98 of the ablation device. This preferably C-shaped component extends partially peripherally around the energy delivery portion 27 to shield the rail device 96 from exposure to the ablative energy. Depending upon the type of ablative energy employed, the material or structure of the directive component 73 can be constructed as set forth above.

To assure the directional position and orientation of the window portion 58 of the ablative device toward the targeted tissue, a key structure 48 is employed. Generally, the transverse cross-sectional dimension of the rail device 96 and matching rail receiving passage 97 is shaped to assure proper directional orientation of the ablative energy. Examples of such key forms are shown in FIGURES 20A-20B.

As with the previous embodiments, the open window embodiment and the rail system embodiment may employ multiple ablative element technology. These include microwave, radiofrequency, laser, ultrasound and cryogenic energy sources.

In accordance with another aspect of the present invention, the tissue ablation system further includes a temperature sensor which is applied to measure the temperature of the ablated tissue during the ablation. In one embodiment, the temperature sensor is mounted to the ablation device proximate the energy delivery portion 27 so that the sensor moves together with the energy delivery portion as it is advanced through the ablation sheath. In another embodiment, the temperature sensor is attached on the ablation sheath.

To determine the temperature of the ablated tissue, a mathematical relationship is used to calculate the tissue temperature from the measured temperature. Typical of such temperature sensors include a metallic temperature sensor, a thermocouple, a thermistor, or a non-metallic temperature sensor such as fiber optic temperature sensor.

In accordance with the present invention, the guide sheath 52 and the ablation sheath 22 can be designed and configured to steer the ablative device along any three dimensional path. Thus, the tissue ablation system of present invention may be adapted for an abundance of uses. For instance, the distal end portion of the ablation sheath can be configured to form a closed ablation path for the ablation device. This design may be employed to ablate around an ostium of an organ, or to electrically isolate one or several pulmonary veins to treat atrial fibrillation. A closed ablation path may also utilized to ablate around an aneurysm, such as a cardiac aneurysm or tumor, or any kink of tumor. In other example, the ablation sheath can be inserted in an organ in order to ablate a deep tumor or to perform any surgical treatment where a tissue ablation is required.

In other instances, the distal end portion of the ablation sheath 22 may define a rectilinear or curvilinear open ablation path for the ablation device. Such open ablation paths may be applied to ablate on the isthmus between the inferior caval vein (IVC) and the tricuspid valve (TV), to treat regular flutter, or to generate a lesion between the IVC and the SVC, to avoid macro-reentry circuits in the right atrium. Other similar ablation lesions can be formed between: any of the pulmonary vein ostium to treat atrial fibrillation; the mitral valve and one of the pulmonary veins to avoid macro-reentry circuit around the pulmonary veins in the left atrium; and the left appendage and one of the pulmonary veins to avoid macro-reentry circuit around the pulmonary veins in the left atrium.

The ablation apparatus may be applied through several techniques. By way of example, the ablation apparatus may be inserted into the coronary circulation to produce strategic lesions along the endocardium of the cardiac chambers (i.e., the left atrium, the right atrium, the left ventricle or the right ventricle). Alternatively, the ablation apparatus may be inserted through the chest to produce epicardial lesions on the heart. This insertion may be performed through open surgery techniques, such as by a sternotomy or a thoracotomy, or through minimally invasive techniques, applying a cannula and an endoscope to visualize the location of the ablation apparatus during a surgery.

The ablation apparatus is also suitable for open surgery applications such as ablating the exterior surfaces of an organ as well, such as the heart, brain, stomach, esophagus, intestine, uterus, liver, pancreas, spleen, kidney or prostate. The present invention may also be applied to ablate the inside wall of hollow organs, such as heart, stomach, esophagus, intestine, uterus, bladder or vagina. When the hollow organ contains bodily fluid, the penetration port formed in the organ by the ablation device must be sealed to avoid a substantial loss of this fluid. By way of example, the seal may be formed by a purse string, a biocompatible glue or by other conventional sealing devices.

As mentioned, the present invention may be applied in an intra-coronary configuration where the ablation device is used to isolate the pulmonary vein from the left atrium. FIGURE 2C illustrates that a distal end of the ablation sheath 22 is adapted for insertion into the pulmonary vein. In this embodiment, the distal end of the ablation device may include at least one electrode used to assess the electrical isolation of the vein. This is performed by pacing the distal electrode to "capture" the heart. If pacing captures the heart, the vein is not yet electrically isolated, while, if the heart cannot be captured, the pulmonary vein is electrically isolated from the left atrium. As an example, a closed annular ablation on the posterior wall of the left atrium around the ostium of the pulmonary vein by applying the pigtail ablation sheath 22 of FIGURES 2 and 4.

In yet another configuration, the ablation device may include a lumen to inject a contrasting agent into the organ. For instance, the contrasting agent facilitates visualization of the pulmonary vein anatomy with a regular angiogram technique. This is important for an intra-coronary procedure since fluoroscopy is used in this technique. The premise, of course, is to visualize the shape and the distal extremity of the sheaths, as well as the proximal and distal part of the sliding energy delivery portion during an ablative procedure under fluoroscopy. It is essential for the electrophysiologist to be able to identify not only the ablative element but also the path that the ablation sheath will provide to guide the energy delivery portion 27 therealong.

Another visualization technique may be to employ a plurality of radio-opaque markers spaced-apart along the guide sheath to facilitate location and the shape thereof. By applying the radio-opaque element that will show the shape of the sheath. This element can be a metallic ring or soldering such as platinum which is biocompatible and very radio-opaque. Another example of a radio-opaque element would be the application of a radio-opaque polymer such as a beryllium loaded material. Similarly, radio-opaque markers may be disposed along the proximal, middle and distal ends of the energy delivery portion 27 to facilitate the visualization and the location of the energy delivery portion when the procedure is performed under fluoroscopy.

To facilitate identification of the distal end portion of the ablation sheath, a fluoro-opaque element may be placed at the distal extremity. Another implementation of this concept would be to have different opacities for the ablation sheath and the energy delivery portion 27. For example, the energy delivery portion may be more opaque than that of the ablation sheath, and the ablation sheath may be more opaque than the transseptal sheath, when the latter is used.

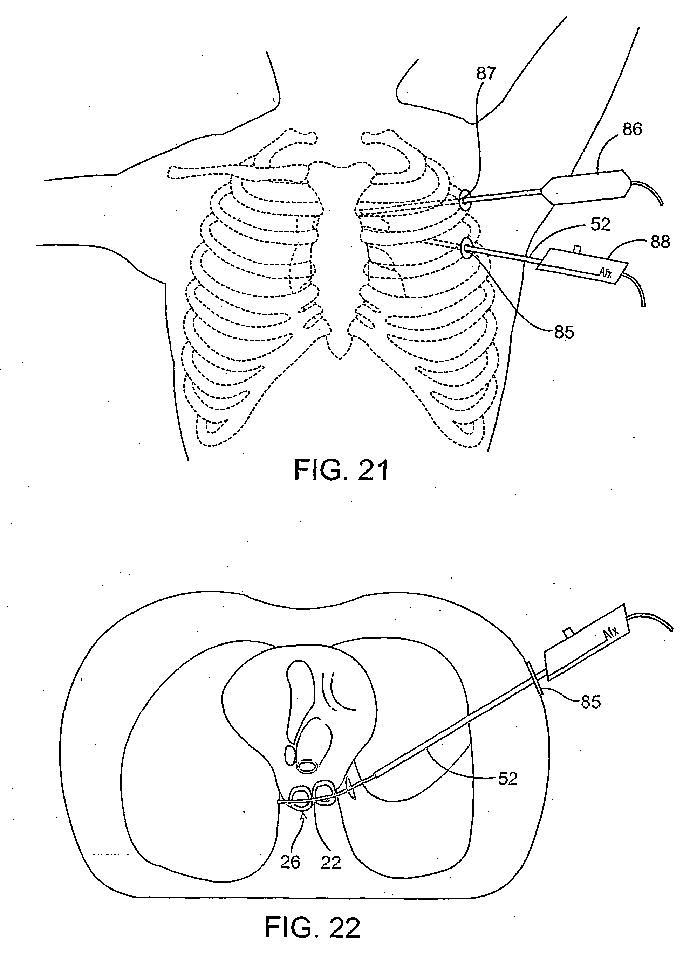

The surgical ablation device of the present invention may also be applied minimally invasively to ablate the epicardium of a beating heart through an endoscopic procedure. As view in FIGURES 21 and 22, at least one intercostal port 85 or access port is formed in the thorax. A dissection tool (not shown) or the like may be utilized to facilitate access the pericardial cavity. For instance, the pericardium may be dissected to enable access to the epicardium of a beating heart. The pericardial reflections may be dissected in order to allow the positioning of the ablation device 26 around the pulmonary veins. Another dissection tool (not shown) may also be utilized to puncture the pericardial reflection located in proximity to a pulmonary vein. After the puncture of the pericardial reflection, the ablation sheath can be positioned around one, or more than one pulmonary veins, in order to produce the ablation pattern used to treat the arrhythmia, atrial fibrillation in particular.

For example, a guide sheath 52 may be inserted through the access port 85 while visualizing the insertion process with an endoscopic device 86 positioned in another access port 87. Once the guide sheath 52 is properly positioned by handle 88, the ablation sheath 22 may be inserted through the guide sheath, while again visualizing the insertion process with the endoscopic system to position the ablation sheath on the targeted tissue to ablate. The ablation device may then be slid through the ablation lumen of the ablation sheath and adjacent the targeted tissue. Similar to the previous ablation techniques, the ablative element of the ablation device may be operated and negotiated in an overlapping manner to form a gap free lesion or a plurality of independent lesions. The ablation sheath may also be malleable or flexible. The surgeon can use a surgical instrument, like a forceps, to manipulate, bend and position the ablation sheath.

The guide sheath, ablation sheath, or ablation element could be controlled by a robot during a robotic minimally invasive surgical procedure. The robot could telescopically translate or rotate the guide sheath, the ablation sheath, or the ablation element in order to position the ablation sheath and the ablation element correctly to produce the ablation of tissue. The robot could also perform other tasks to facilitate the access of the ablation sheath to the tissue to be ablated. These tasks include, but are not limited to: performing the pericardial reflection in the area of a pulmonary vein; performing an incision on the pericardial sac; manipulating, bending or shaping the ablation sheath; or performing an incision on an organ to penetrate the ablation sheath through the penetration hole.

In accordance with yet another aspect of the present invention, the concept of using a sliding ablation element in an ablation sheath to ablate from the epicardium of a beating heart can also be applied in open chest surgery. In this procedure, a malleable ablation sheath may be beneficial, as compared to a pre-shaped ablation sheath. For example, a malleable metallic wire (e.g., copper, stainless steel, etc...) could be integrated into the ablation sheath. The cardiac surgeon will then shape the ablation sheath to create the ablation path that he wants and will finally produce the ablation line by overlapping several ablations

In this technique, it is important to note that the ablation sheath must be stabilized against the epicardium since the ablation sheath will define the ablation path of the energy delivery portion. Should the ablation sheath be inadvertently move during the process, the final ablation line may be undesirably discontinuous. Thus, a securing device may be applied to secure the ablation sheath against the epicardium. Such a securing device may include stitches or the like which may be strung through receiving holes or cracks placed in the ablation sheath. Another device to anchor the ablation sheath to the epicardium may be in the form of a biocompatible adhesive, or a suction device.

In accordance with yet another aspect of the present invention, a way to visually locate the ablation element within the ablation sheath is provided to the surgeon. In one embodiment of the invention, the ablation sheath is transparent and the ablation element can be directly visualized, or indirectly visualized via an endoscope. In yet another embodiment of the application, a marking element that can be directly visually identify along the ablation sheath, or indirectly visualized via an endoscope, is used to identify the location of the ablation element within the sheath. The marking element is sliding with the ablation element to show the location of the ablation element.

In accordance with yet another aspect of the present invention, a way to indirectly locate the ablation element within the ablation sheath is provided to the surgeon. A position finding system is incorporated in the handle of the device to indicate the position of the ablation element within the ablation sheath. At least one marker can be directly visually, or indirectly visually identified. These markers can be used in collaboration with the position finding system as reference points to identify the location of the ablation element.

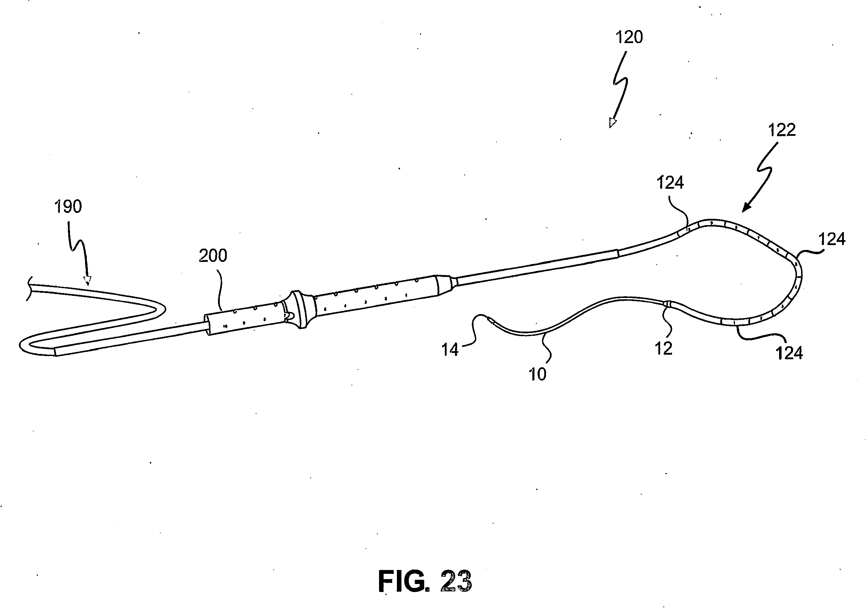

Now turning to FIGURE 23, another embodiment in accordance with the present invention will be discussed. More specifically, FIGURE 23 depicts ablation system 120 generally comprising an energy transmission conduit 190, a handle portion 200, an ablation sheath 122, and a distal end portion 10. In the following discussion and associated figures with respect to ablation system 120, like reference symbols (offset by 100) refer to like parts with respect to ablation system 20.

End portion 10 is adapted to better guide the sheath 122 in the proper orientation adjacent or proximate to the target tissue to be ablated. Portion 10 can be formed in any suitable fashion to facilitate proper placement of sheath 122. Portion 10 is constructed from any suitable material allowing the necessary flexibility to be guided through tight areas with a human body, around the pulmonary veins between the pericardium and the epicardial tissue of the heart for example. While portion 10 may be made from any suitable material, silicone, expanded PTFE, Pebax, or polyurethane for example, portion 10 is preferably constructed from porous expanded PTFE. Portion 10 may be placed within tubular member 12, providing portion 10 greater lateral force allowing a user to more easily direct, or otherwise guide, the portion 10 in the body cavity. As should be readily apparent, portion 10 may be of any suitable length for a given ablation procedure. For example, portion 10 may be made having a suitable length to encircle a body organ, the heart for example, such that the ablation sheath is not specifically manipulated during initial placement of portion 10. In this way, undesirable damage to portion 10 is minimized.

As shown, portion 10 may terminate in a blunt end 14 as a means to protect surrounding tissues during the guidance process. Blunt end 14 may comprise suture (not shown) which may further help users in guiding portion 10. Suture lines may be grasped by medical tools or instruments for example, allowing a user to more easily place portion 10. For illustration purposes only, a user can blindly advance the distal end of portion 10 around a bodily object until the sutures once again appear. The medical tool may then be used to grasp the distal end of the portion 10 allowing for the complete encircling of the object by the guide portion 10 and, ultimately, the ablation sheath. Such a tool is described in commonly assigned U.S. Patent No 6,802,840.

Guiding portion 10 is radially sized to allow for guiding through narrow openings naturally occurring or created by the user during use. If portion 10 is radially sized different than the ablation sheath 122, an enlarger/reducer may be utilized to facilitate the transition from the sheath 122 to portion 10. For example, if the guiding portion 10 is radially sized smaller than the ablation sheath, as generally shown in FIGURE 23, reducer 12 may be utilized to facilitate the transition from the larger diameter sheath to the smaller diameter portion 10. As with the blunt end 14, the reducer 12 may be made from any suitable material, stainless steel or thermoplastic elastomer for example.

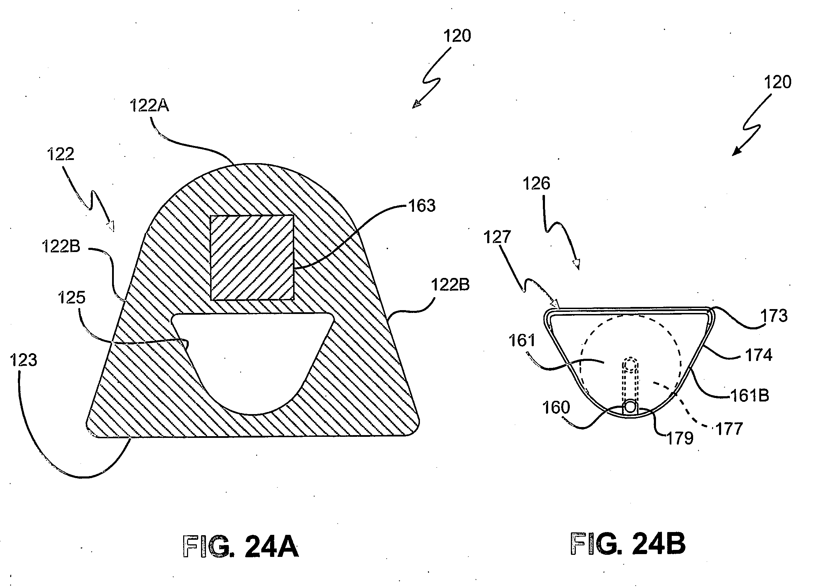

Now turning to FIGURE 24A, ablation sheath 122 as part of system 120 will be described. Sheath 122 is generally functionally similar to sheath 22. For example, both ablation sheath 22 and ablation sheath 122 generally act to define an ablation path about which the ablation device is guided.

As stated above, ablation system 120 comprises an elongated ablation sheath 122 having a cross-section as generally shown. Sheath 122 may be constructed from any suitable material. Preferably sheath 122 is formed from porous expanded PTFE, which provides the requisite flexibility to pass through various body cavities or take on curvatures associated with various body organs, the heart for example, and superior radial strength, resistant to lateral compression such that the translation of an ablative element therethrough is not compromised. Such a material also has the advantage of improved torque, such that when a user rotates a portion of the material external to the patient's body, the remaining portion within the body is encouraged to rotate into the same orientation. While such a material is not required, it is important to note that any material having at least these characteristics is considered to be within the bounds of the present invention.

The longitudinal geometric shape of sheath 122 is adapted to encourage proper orientation.of the sheath with respect to the target Tissue. Additionally, the geometric shape provides enhanced thermal protection with respect to tissue adjacent or in the general vicinity of the target tissue.

While sheath 122 may be adapted to any suitable cross-sectional shape which provides all or any combination of the benefits described herein, sheath 122 preferably comprises a curvilinear top portion 122A, angled side portions 122B and a substantially flat bottom portion. It is important to note that, while a specific dimensional relationship between the sides of the geometric cross-section is not a requirement, the relationship shown in FIGURE 24A has particular advantages, as will become apparent in the discussion below.

Top portion 122A. as shown, is generally circular allowing for dynamic contact with surrounding tissues while minimizing undesirable damage based on such contact. Bottom portion 122C is suitably dimensioned to encourage the orientation of sheath 122 such that contact between the generally flat portion 123, also referred to as the active ablation side, and the targeted biological tissue is maintained during use. To further limit collateral tissue damage, flat portion 123 interfaces with side portions 122B via a curvilinear section, as shown.

Referring also to FIGURE 24B, sheath 122 further comprises ablation lumen 125 adapted to slideably receive the ablation device 126 of FIGURE 24B. Lumen 125 is shaped having a substantially flat top surface and a substantially curvilinear bottom surface encouraging/maintaining proper orientation of ablation device 126 translating therein, as will be discussed in more detail below. While lumen 125 is defined to have a specific shape, it should be apparent that any suitable geometric shape which results in minimizing the rotation of ablation device 126 within lumen 125 is sufficient. Such shapes, for example, may have at least one cross-sectional side which is not curved. For illustration purposes only, the cross-sectional geometry could be in the shape of a "T" where the antenna is located at the base of the "T". Additionally, it should be noted that any given geometric cross-section which minimizes angular rotation about the longitudinal axis of the device 126 is within the contemplation of the present invention.

As with sheath 22, sheath 122 may further comprise a retaining member 163. Member 163, as with sheath 22, may be malleable allowing the user to conform the sheath to a specific orientation for use. Alternatively, member 163 may be adapted to retain the specific length of sheath 122 such that a user can be assured of the position of the ablation device translating therein. For example, if sheath 122 is constructed from porous expanded PTFE, a material which offers tremendous resistance to radial compression, however, can longitudinally expand and contract, member 163 would act to limit the longitudinal expansion and contraction of the sheath 122 over its length. For such cases, a member 163 constructed from a malleable material may provide this function. For cases where a more flexible sheath 122 is desired, member 163 may be constructed from a more flexible material, such as silicone, or plastics or metals which have the desired degree of flexibility, for example. It should be noted that the member 163 may be adapted to having a differing resistance along its length. Furthermore, this differing resistance may also be coupled with varying flexibility of member 163 about its length, the resulting element specifically designed or encouraged to take on more specific shapes when deployed. For example, to encourage bending at a given point along the length of member 163, its flexibility and/or its resistance to expansion and contraction forces at the point of curvature may be less than at other locations about member 163.

Alternatively, a combination of elements may be employed. If, for example, one element offers a high resistance to expansion forces but is compressible, such an element can be combined with another element which offers a higher degree of resistance to compression forces, the combination providing the desired sheath 122 characteristics. It should be noted that a plurality of elements having differing degrees of resistance can be combined.

Additionally, member 163 may be formed by any material described herein which acts to further thermally isolate tissue adjacent to the target tissue being ablated, tissue adjacent to side portions 122A and 122B for example. Use of such a material for member 163, alone or in combination with other materials having differing characteristics as described immediately above, may assist in the reduction of the overall geometric characteristics of the cross-section of sheath 122, allowing access to correspondingly smaller areas within the patient's body. Additionally, it should be readily apparent that such a material having good thermal isolating characteristics may also have the desired expansion/contraction characteristics. For a given procedure, silicone may be such a material for example.

In some cases it may be desirable to adapt at least a portion of the external surface of sheath 122 in such a manner as to make that portion non-permeable. For the purpose of this discussion, non-permeable shall mean that fluids, including gases, are substantially prevented from passing a point external to the sheath 122 outer surface to a point within the ablation lumen 125.

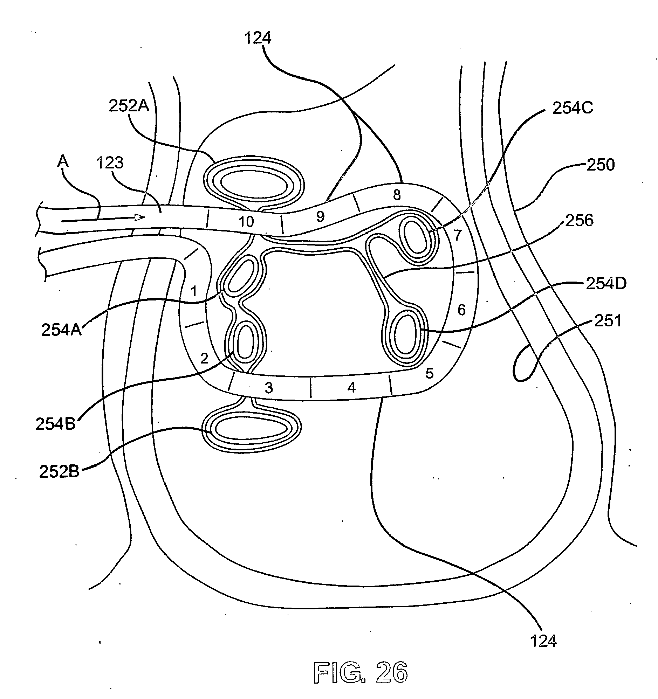

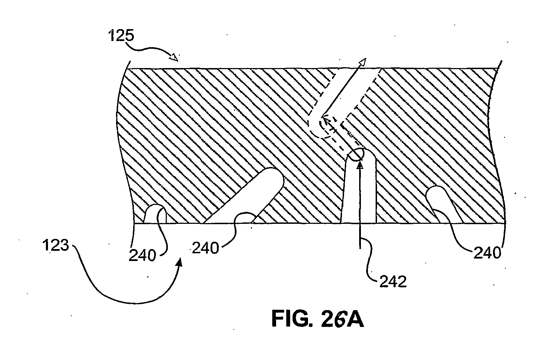

Now referring to FIGURE 26A-C, the process of adapting the sheath 122 will be discussed in greater detail. As shown in FIGURE 26A, the surface of expanded PTFE may be porous to the extent that fluids can pass completely therethrough. This can be disadvantageous since such fluids can interact with the ablation system, impairing the ability of the system to transmit ablative energy therefrom. It is important to note that the surface characteristics of expanded PTFE, as part of sheath 122, have been simplified for the sake of discussion.

As shown in FIGURE 26A, the sheath comprises networks of channels 240, which may, through the manufacturing process, provide one or more passageways 242 which would provide fluid communication between the external surface of sheath 122 and lumen 125. As stated above, the structure of the porous expanded PTFE is shown in a simplified manner, and many more passageways 242 may exist.

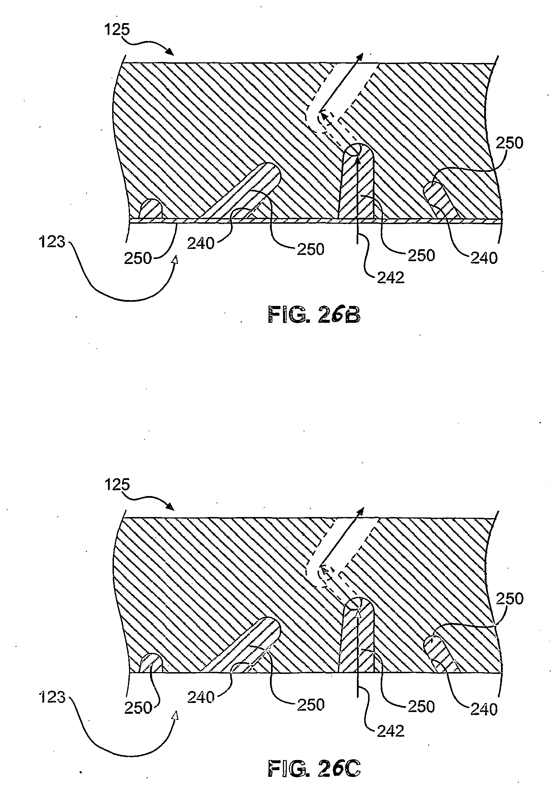

With reference also made to FIGURE 26B, the external surface of sheath 122 may be adapted to prevent fluid communication via passageways 242. According to the invention, the porous expanded PTFE of sheath 122 is made partially or substantially impervious to fluid ingress by filling the openings 240 near the external surface of sheath 122. Depending upon the desired flexibility at the point of adaptation along sheath 122, differing materials may be used as filler material. If, for example, flexibility is desired, an elastomeric material, silicone or other suitable material having similar flexibility and electrical isolating properties for example, may be utilized. In this way, the sheath 122 may be made impervious to fluid ingress at the points of adaptation without compromising the flexibility at these points. It is important to note that it may be desirable to not adapt certain sections of sheath 122, allowing for placement of certain fluids, such as saline or conductive fluids for ablation systems which rely on modalities different than microwave energy, or certain drugs which would assist in the natural healing process subsequent to ablation, the drugs adapted to pass through the openings of the expanded PTFE.

On the other hand, if you would like to make that point of sheath 122 less flexible, as is discussed elsewhere, other less flexible materials or rigid materials may be utilized. As should be readily apparent, different lengths of sheath 122 along its longitudinal axis may be made having differing levels of flexibility by simply impregnating the openings with materials of different levels of flexibility. Such a sheath would be adapted to encourage use of the ablation system for more specific ablation applications, positioning around the pulmonary veins and upon the epicardial surface for example. It should be noted that such impregnating materials should be consistent with the ablative energy utilized by the ablation system.

The impregnating or filler material may be applied to the sheath in any suitable manner, such as dip coating or direct application for example. During application, filler material 250, silicone for example, fills openings 240 and a thin layer of material 250 is deposited on the exterior surface of sheath 122 as generally shown in FIGURE 26B. While the deposited material 250 can define the external surface of the sheath 122, it is desirable to trim the material 250 from the external surface of the sheath 122, for example from side 123 of sheath 122, to decrease the distance between the ablating device and the tissue, improving the effectiveness of the ablation system. In such a case, after the impregnating material is deposited upon the external surface and into the openings 240 of the sheath, the external layer 250A can be removed or peeled away providing a consistently smooth non-permeable surface 252 as shown in FIGURE 26C.

It should be noted that material impregnation can be limited to a radial portion of the sheath, as well as along different longitudinal lengths as discussed above. For example, for procedures upon the epicardial surface of a heart, material impregnation may only be desired on the side 123 in contact with the tissue to be ablated. In this case, the sheath may be horizontally dipped into a bath of material, silicone for example, to a desired depth corresponding to the desired amount of coverage. Alternatively, the silicone material may be applied directly to side 123 through the use of a transfer wheel, the bottom portion of the wheel being partially submerged in a silicone bath. With such a system, the wheel would be made to rotate and the sheath side 123 would be translated upon the top of the wheel, the rotational movement of the wheel acting to pick up silicone from the bath and transferring it along side 123 of sheath 122. The wheel is preferably rotated in a direction opposite to the translation of side 123.

Referring now to FIGURE 24B, ablation device 126 comprises an energy delivery portion 127 which comprises an insulator 161 and director component or reflector 173. As with insulator 61, insulator 161 is preferably provided by a good, low-loss dielectric material which is relatively unaffected by microwave exposure, and thus capable of transmission of the electromagnetic field therethrough. Moreover, the insulator material preferably has a low water absorption so that it is not itself heated by the microwave energy. Insulator 161, as with insulator 61, may be constructed from any suitable materials including, but not limited to, moldable TEFLON^{®}, silicone, or polyethylene, polyimide, porous expanded PTFE, etc.

Sheath 122 further comprises a plurality of position indicators 124 along a portion of its length, as generally shown in FIGURE 23. These indicators 124 define one or more positions along the sheath 122 within which the energy delivery portion 127 travels. The plurality of position indicators 124 cooperate with other control and/or feedback systems as part of system 120, as described in greater detail below, to enable a user to know the exact position of the energy delivery portion 127 translating within the sheath 122 at any time during use.

While the indicators 124 of FIGURE 23 are shown comprising numerical values as identifiers, any suitable means of identifying or distinguishing the plurality of positions may be used. For example, roman numerals, or other alphanumeric or graphical symbols may be used. As shown in FIGURE 23, indicators 124 may further comprise graphical indicators which more precisely define the plurality of positions.

The indicators 124 may be incorporated into system 120 in any suitable means. For example, indicators 124 may be defined through an inking process, providing a contrast between indicators 124 and sheath 122. Alternatively, indicators 124 may also comprise various materials which fluoresce, allowing the user to view the sheath positions 124 on a monitor (not shown) as part of a fluoroscopy system of system 120, for example. In systems incorporating indicators 124 which fluoresce, the indicators 124 may further be adapted to indicate rotation orientation of sheath 122 as well.

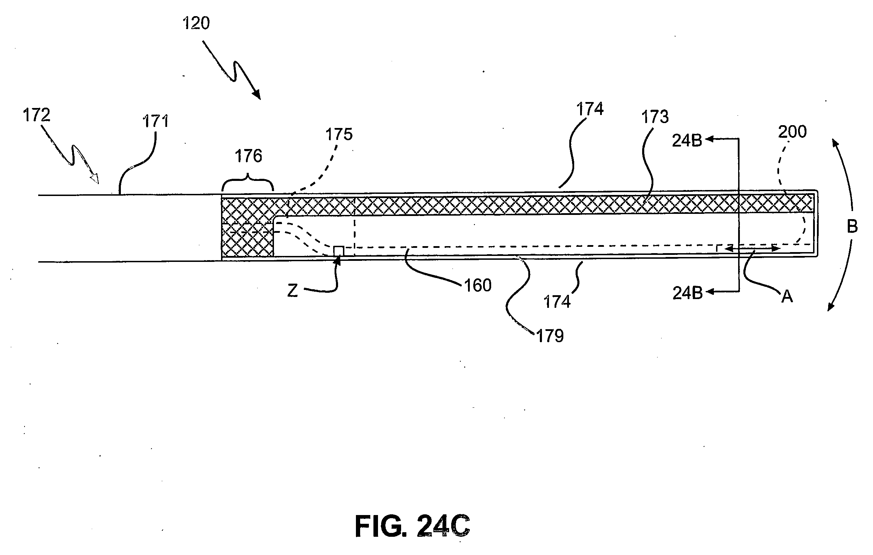

Referring also to FIGURE 24C, insulator 161 comprises a longitudinal groove 179 adapted to receive antenna 160 therein. Groove 179 also acts to restrict the lateral movement of antenna 161 during use, keeping the antenna 160 fixed with respect to reflector 173 during use for example. As the ablation device deflects during use, as generally indicated by arrows B for example, groove 179 allows the antenna to translate therein as indicated by arrows A. While arrows B indicate deflection in one plane, this is done for explanation purposes only. It should be apparent that the flexible ablation device 126 can deflect in any desirable direction during use. During such deflection, groove 179 acts to restrict lateral movement of the antenna 161. With respect to the longitudinal axis of the energy delivery portion 127, it's important to note that while the flexible ablation device can deflect in any desirable manner, insulator 161 maintains the proper orientation of reflection 172 with respect to antenna 160, as does insulator 61 with respect to antenna 60.

Reflector 173 may be constructed or configured in any manner consistent with reflector 73 described herein. Flexible reflector 173 may be constructed from copper mesh, for example, adapted to restrict the passage of electromagnetic energy therethrough while allowing for the desired flexibility. Such a mesh would have crossing wire elements defining openings therebetween which are sized to limit the passing of microwave energy at the desired frequencies described herein.

Alternatively, reflector may be constructed from a thin flexible metallic, or otherwise electrically conductive, film. Whether mesh or a solid conductor, reflector 173 is most preferably flexible enough to allow the deflection of energy delivery device 127 in all directions while restricting the passage of electromagnetic energy therethrough.

Flexible reflector 173, as depicted in FIGURES 24B-C, is attached to the top surface of energy delivery portion 127 using any suitable means, such as epoxy bonding for example. Alternatively, reflector 173 may be press fit or otherwise formed atop insulator 161. A thin insulating material 174, preferable shrink tubing or the like, is applied over the entire surface of the energy delivery portion 127, holding the reflector 173 and the insulator 161 in place with respect to each other. As should be readily understood, insulator 174 also acts to retain antenna 160 within groove 179. Such use of insulator 174 allows for closer placement of antenna 160 tot eh active ablation side 123 of sheath 122.

As shown, the longitudinal sides of reflector 173 preferable curve over the top surface 161A and follow side portions 161B of insulator 161 resulting in a curvilinear cross-sectional geometry more suitable for directing at least a portion of the electromagnetic energy produced by antenna 161, towards flat bottom portion 123 of sheath 122, and the target tissue during use.

Preferably, reflector 173 is electrically connected to the energy transmission conduit 190, a transmission line 172, similar to transmission line 72. Transmission line 172 comprises an outer covering 171, an inner conductor 175, an outer conductor 176 and an insulator therebetween 177.

More specifically, reflector 173 is connected to outer conductor 176 of transmission line 172 using any suitable means. For example, as specifically depicted in FIGURES 24B-C, reflector 173 is constructed from a generally rectangular piece of material. The proximal end of the rectangular piece may be formed in a cylindrical manner generally taking on the cylindrical shape of outer conductor 176. The reflector 173 may be attached to outer conductor 176 using any suitable means allowing for the flexibility of system 120. While the connection may be made via a solder joint or a compressible sleeve (not shown) which can be mechanically compressed or crimped, operably connecting the reflector 173 to outer conductor 176, the connection is preferably made using an epoxy, or other adhesive, which better preserves the flexibility. The remaining proximal portion may be trimmed as necessary to create the desired cross-sectional geometric shape to achieve the unidirectional characteristics desired. For example, the reflector may be adapted to further limit or focus the electromagnetic energy as desired. It is important to note that while the reflector 173 is described as having a curvilinear cross-sectional geometric shape, if a wider lesion is desired, a more planar shaped reflector atop surface 161A would result in such a lesion and is contemplated in accordance with the invention disclosed herein.

As should be readily understood, the shield or reflector contour may be defined simply by adapting insulator 161 with the desired surface upon which the reflection 173 is applied using any means disclosed herein.

Additionally, it should be noted that reflector 173, as with reflector 73, has two main functions: to prevent the passage of ablative energy therethrough and to reflect at least a portion of the ablative energy in a desired predetermined direction, therefore, restricting the passage of ablative energy therethrough and protecting tissue in the vicinity of the target tissue during an ablation procedure.

While flexible reflector 173 is shown attached atop the top flat surface of energy delivery device 127, reflector 173, as with reflector 73, may be embedded within insulator 161. As with the reflector 173, the antenna 160 may be mounted within insulator 161 as disclosed herein with respect to antenna 60 and insulator 61.

As shown in FIGURE 24C and in a similar fashion as ablation system 20, center conductor 175 is generally in line with the longitudinal axis of energy delivery portion 127 while the antenna portion 160 is offset from the longitudinal axis. While not necessary in practicing the disclosed invention, the offset nature of the antenna with respect to the longitudinal axis of the energy delivery portion 127 allows for the deposition of a greater amount of ablative energy within the target tissue. While the antenna portion 160 is preferably closer to the target tissue, direct contact with the target tissue is undesirable since, during use, the antenna structure may become heated and such a heat source may lead to charring or otherwise undesirable and unwanted tissue damage. Such additional heat sources may also result in uneven tissue ablation with respect to tissue depth requiring the premature removal of the ablation device from the target tissue, preventing transmural lesion creation. Such problems are typically associated with ablation systems requiring direct contact with the target tissue, such as RF electrode, electrical resistive heating or cryogenic ablation systems.

As shown in FIGURE 24C, antenna 160 may be electrically interfaced to center conductor 175 through an impedance Z. Impedance Z can be inductive or capacitive in nature and acts to electrically tune the system 120, maximizing energy passage from the ablative energy source to the ablative element, in this case microwave energy delivered to antenna 160. The system 120 is tuned such that maximum energy is transferred to the target tissue when the active surface, surface 123 of sheath 122 for example, is placed upon the target tissue.

In operation, as with energy delivery portion 26, energy delivery portion 126 is placed into and translates within ablation lumen 125. As noted above, the cross-sectional geometric shape of the energy delivery portion 126 and lumen 125 are adapted to minimize rotation of portion 126 with respect to sheath 122.

As will be discussed in more detail below, antenna 160 is translated by activation of an actuator ring 211 as part of handle 200. Antenna 160 may be any suitable length compatible with the ablative energy source utilized. For example, differing frequencies of microwave energy generally require correspondingly differing antenna lengths. Additionally, it is important to note that impedance Z may be manipulated to allow a non-standard antenna length for a given frequency. It should be apparent, therefore, that different antenna' lengths are achievable. Since antenna length is directly proportional to the lesion created, some antenna lengths may be more desirable for specific tissue ablation procedures.

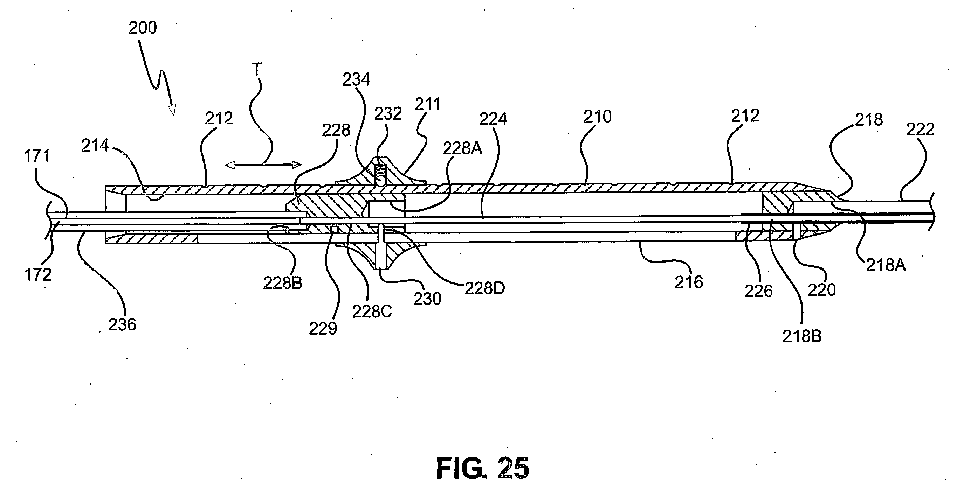

Now turning also to FIGURE 25, the handle portion 200 will be described in greater detail. It will become apparent from the following discussion that the design of certain geometric features of handle 200 are directly related to the characteristics of the ablation device used. It should be noted, therefore, that geometric features differing from those specifically discussed herein are considered within the bounds of the present invention.

Handle 200 comprises a handle body 210, an actuator ring 211, a piston 228, a barrel 218 and an inter and an outer telescoping tube, 224 and 226 respectively. Body 210 comprises a means for positioning the energy delivery device 127 within sheath 122 one of the desired plurality of positions indicated by position indicators 124, as discussed above. As depicted, the positioning means may comprise a plurality of indentions 212 adapted to provide feedback to the user when the ablation element is properly placed. Indentions 212 provide tactile feedback indicating the current position or placement of the energy delivery portion 127 within sheath 122, as is discussed in more detail below. Indentions 212 are adapted to cooperate with a ball 234 and spring 232 arrangement as part of actuator ring 211. As actuator ring 211 is translated about handle 200, force of spring 232 encourages the ball 234 to partially engage one of the plurality of indentions 212.

While discussed in terms of the operative combination of indentions 212, spring 232 and ball 234, the user feedback can be provided using any other suitable means. For example, partial or total circumferential ridges positioned along the outer surface of body 210, at locations similar to indentions 212, may be adapted to engage a corresponding indention as part of the inner surface of ring 211, for example at a location consistent with ball 234. Alternatively, the ridge can be part of the ring 211, the ridge adapted to engage one of a plurality of corresponding indentions along the handle body 210. Additionally, positioning means 212 further comprises identifiers which indicate the position of energy delivery device 127 within sheath 122 at any given indention 212.

Further, it should be readily understood that the user feedback may be provided by other means, such as audible or visual indications, in lieu of tactile means. For example, system 120 could comprise elements which produce audible sounds indicating the position of energy delivery device 127. The audible sounds may be in any form suitable for and consistent with such indications, such as electronically producing the alphanumerical representation of the position or providing a series of sounds which change in pitch or amplitude as the ring 211 translates from one end of the handle body 210 to the other.

Visual indications may be provided in any suitable manner, either locally, as part of handle 210 for example, or more remotely as a display as part of system 120. For illustrative purposes only, ring 211 may comprise an illuminating device, a light emitting diode or the like for example, which illuminates at one or more positions along the longitudinal length of handle 200 to dynamically indicate the position of energy delivery device 127. The visual means may comprise a plurality of illuminating elements, one for each position for example, or one multi-colored element may be used, each color representing a different position. Alternatively, a single element may be used, the element flashing to indicate position. For example, the element could flash a predetermined number of times corresponding to the current position. The flashing could be repeated after a short delay to give the user the ability to easily decode the flashing signals. For illustration purposes only, the element could flash once for position 1, twice for position 2 and *n* times for position *n*, where *n* is the total number of positions.

Body 210 further comprises a bore 214 and a longitudinal slot 216. While bore 214 is shown as cylindrical in shape, any suitable geometric shape may be used, geometric shapes having a plurality of longitudinal sides for example may be used. Such shapes also help to reduce rotation of piston 228 within body 210 reducing undesirable torsion forces on the sheath 122 and/or ablative device 127 therein.

Piton 228 comprises a distal bore 228A, a proximal bore 228B, and a channel 228C therebetween. Proximal bore 228B is sized to accept a flexible tube 236. Flexible tube 236 acts to provide strain relief to the transmission cable 172 therein. Tube 236 may be made from any suitable material, such as silicone tubing, and may be attached in any manner compatible with piston 228. For example, tube 236 may be compress fit into bore 228B or may be held within bore 228B through application of an adhesive.

Inner telescoping tube 224 is compress fit into, or otherwise held within, bore 228C. Prior to insertion of tube 224 into channel 228C, tube 224 is crimped onto transmission line 172 placed therein. As will be made apparent through the discussion below, the combination of inter and outer telescoping tubes 224, 226 provide greater longitudinal force required to translate transmission line 172 within sheath 122.

While tube 224 may be crimped to the transmission line in any suitable fashion, preferably, tube 224 is crimped onto line 172 with a crimp having a triangular geometric cross-section, encouraging equal compression forces about the line 172 while providing several raised points engaging and holding tube 224 within channel 228C. Most preferably, since radial compression of transmission line 172 may impact its ability to most effectively transmit ablative energy to the ablative element, compression of the tube 224 upon line 172 is achieved through crimping tube 224 to substantially only the outer covering 171. The various ridges produced during the crimping process also act to better seat tube 224 within channel 228C. If necessary, a set screw 229 may be utilized, alone or in addition to the crimp, to hold tube 224 in place relative to piston 228. It is important, however, to limit the compression of the transmission line 172 since such compression may impair efficient energy transfer to the ablation device.

Distal bore 228A comprises a radial bore 228D sized to accept the distal tip of an actuator pin 230, as shown. The proximal portion of pin 230 is held in place in actuator ring 211 in any reasonable manner. For example, pin 230 may be held in place through the use of adhesives or may be compress fit. Alternatively, pin 230 may comprise threads which engage corresponding threads on ring 211, pin 230 being screwed into and held in place within ring 211. As shown, a portion of the tip of pin 230 rides within longitudinal groove 216. Groove 216 has a width equal to the diameter of the distal portion of pin 230, limiting rotational movement of the actuator ring 211/piston 228 assembly as they translate about handle 200.

Barrel 218 is held within bore 214 with a pin 220 which passes through corresponding openings in handle body 210 and barrel 218, as shown. As with other elements described herein regarding handle 200, pin 220 may be held in place using any suitable means, such as an adhesive. As with pin 230, pin 220 may be threaded. Alternatively, pine 220 may be a spring pin having a longitudinal opening its entire length and an uncompressed diameter slightly larger than the corresponding openings in the body 212 and barrel 218. Thus, once compressed and placed within the openings, upon removal of the compression forces, pin 220 is held in place by the force generated by the pin 220 trying to take on its uncompressed form.

Barrel 218 comprises a distal bore 218A and a longitudinal channel 218B, as shown. Channel 218B accommodates outer telescoping tube 226. Outer tube 226 is held within channel 218B using any suitable means disclosed herein, including epoxy bonding. As shown, inner telescoping tube 224 is sized to translate within outer telescoping tube 226. As stated above, the telescoping tubes 224, 226 act to provide transmission line 172 longitudinal support to better facilitate translation of transmission line 172 within sheath 122. Barrel 218 holds tube 226 stationary with respect to handle body 210 while movement of actuator ring results in a coordinated movement of the inner telescoping tube 224, and the transmission line 172 therein, within tube 226.

A tube 222 is mounted to the distal portion of barrel 218, as shown. Tube 222 protects the telescoping tube 226 from forces associated with entering the distal portion of the tube 222 into a patient body, through an access port, for example. Tube 222 is held within bore 218A using any means described herein, such as epoxy bonding. Alternatively, tube 222 may be held in place by a threaded pin 220, described above, which may forcibly engage tube 222, holding barrel 218 and tube 222 stationary with respect to handle body 200.

As shown, telescoping tube 224 is sized to receive transmission line 172, limiting movement of line 172 therein. Similarly, tube 226 is sized to receive tube 224 with minimal play. As stated above, tube 222 acts to protect the telescoping tube 226 from forces applied by a user during use of system 120. (See also FIGURE 23).

As should be readily apparent, the length of telescoping tube 224 is dependent on the length of travel of the tube within handle body 210, preferably providing a closed path from the proximal opening of telescoping tube 224 to the proximal opening of telescoping tube 226. For example, where a long lesion path is desirable, a corresponding long tube 224 is required to provide a closed path for the transmission line 172 from piston 228 to the proximal opening of tube 226.

As should be readily apparent, the length of tube 226 is directly dependent on the length of sheath 22 and the corresponding force needed to translate the ablative device therein. The length of tube 222 is preferably at least the length of tube 226 to properly protect tube 226, as described above.

In operation, indentions 212, as part of the positioning means, are spaced apart along handle 200 a distance substantially equal to the length of the ablation element, antenna 160 for example. More preferably, the spacing is defined as having a length slightly shorter than the length of the ablation element to allow for overlapping of individual lesions during use, resulting in the creation of a continuous long lesion if desired.

Alternatively, the spacing of the indentions 212 may be defined by other parameters or characteristics related to the ablation element utilized. For example, the ablative element may be dynamically adjustable such that the ablative energy emitted therefrom is controlled along the longitudinal length of the element. In such cases the spacing may be defined in terms of at least one desirable length, preferably the minimum length. Additionally, in such cases, system 120 would further comprise means for effecting such a change in length and an indicator used to associate the length of the ablative element to the indention 212 spacing on handle body 210.

With reference also to FIGURE 26, the relationship between the indentions 212 and placement of energy delivery portion 127 can be more readily understood. As stated above with respect to ablation system 20, ablation system 120 may be used to ablate various biological tissues, including epicardial heart tissue around the pulmonary veins.

FIGURE 26 depicts an exemplary placement of ablation sheath 122. For clarity the heart has been removed from a patient's pericardial sac 250, which has been partially removed to show its posterior surface 251. Various vascular landmarks are depicted generally where they would interface to the heart. These include the superior and inferior vena cava, 252A and 252B respectively, and the upper and lower right and left pulmonary veins, 254A-D respectively. Also shown in dissection is the pericardial reflections 256 which accompany the vascular structures.

Generally, sheath 122 of system 120 gains access to the patient's body through any suitable means, through a port access point or other minimally invasive opening for example, and is advanced as indicated generally by arrow A. Advancement of sheath 122 is achieved through devices and methods described herein. For example, portion 10 of system 120 may be utilized to initially define the position of sheath 122. For illustration purposes only, the portion 10 may be initially advanced to a point slightly inferior to the left inferior pulmonary 254D. At this point an additional medical tool may be utilized to grab the sutures of blunt end 14 of portion 10 and retracted therefrom, further guiding, or otherwise positioning, portion 10 about the pulmonary veins 254, as generally depicted.

Also depicted in FIGURE 26, position indicators 124 are shown for illustrative purposes only. As described above, such indicators 124 are typically provided on the top surface 122A, since this surface is more easily viewed during use. Here they are provided as a reference for discussion only.

Once the sheath 122 is properly placed, side 123 of the sheath 122 is positioned to engage the epicardial surface of the patient's heart, the ablation device is then translated therein. Energy periodically and selectively emitted from the ablation device at various positions results in the creation of at least one desired lesion. For example, if a long continuous lesion around the pulmonary veins is desired, the user would translate the ablative device, using methods and devices described herein, pausing to ablate tissue at each position indicator 124, the result being a long continuous and gap-free lesion corresponding to position indicators 124 having position identifiers 1 through 10.

While this illustration depicts ablation around the pulmonary veins, any ablation patterns contemplated may be achieved in a similar manner. Additionally, it should be noted that, while generally described in terms of accessing the pericardial sac 250 from the patient's right side, other access points associated with various procedures, alone or in combination, are also contemplated. Such access points include those associated with, but are not limited to, sternotomy, thoracotomy or thoracoscopy, or xiphoid procedures.

While the ablation system 120 has been specifically described in terms of a microwave based system, it should be readily apparent that any other system based on other energy sources described herein may be adapted to take advantage of one or more characteristics of system 120.

Although the foregoing invention has been described in some detail for purposes of clarity of understanding, it will be apparent that certain changes and modifications may be practiced within the scope of the appended claims.

## Claims

1. An ablation system having a non-permeable portion, comprising:
a handle portion (200) having proximal and distal ends;
a flexible elongated tubular member (122) having at least one lumen (25) therethrough operably attached to the distal end of the handle portion, the flexible elongated tubular member constructed from a porous material;
an energy delivery device (27) comprising at least one ablative element (26), the energy delivery device operably attached to the handle portion and adapted to translate within a first of the at least one lumen of the tubular member;
an ablative energy source operably attached to the at least one ablative element; and
a filler material disposed in openings (240) near the external surface of the tubular member (122) along at least one portion of the tubular member defining an initial adaptation point,
wherein the flexible elongated tubular member is non-permeable to fluids at the adaptation point.

2. The system of claim 1, wherein the initial adaptation point includes the length of the flexible tubular member along which the energy delivery device translates.

3. The system of claim 1, wherein the initial adaptation point includes the entire length of the flexible tubular member.

4. The system of claim 2, wherein the porous material is expanded PTFE.

5. The system of claim 3, wherein the filler material is an elastomer.

6. The system of claim 4, wherein the filler material is silicone,

7. The system of claim 5, wherein the at least one ablative element is adapted to transmit electromagnetic energy and the filler material is transparent to the transmitted electromagnetic energy.

8. The system of claim 2, wherein the filler material is flexible whereby the flexible nature of the tubular member at the initial adaptation point is retained.

9. The system of claim 2, wherein the filler material is not flexible whereby the overall flexibility of the tubular member at the adaptation point is reduced.

10. The system of claim 2, wherein the filler material is rigid, whereby the overall flexibility of the tubular member at the adaptation point is substantially reduced.

11. The system of claim 1, wherein the filler material is deposited at one or more portions of the tubular member, each defining a separate adaptation point.

12. The system of claim 11, wherein the filler material used at each adaptation point is flexible, whereby the overall flexibility of the tubular member remains flexible.

13. The system of claim 11, wherein the filler material used at each adaptation point is predetermined such that the amount of flexibility of the tubular member along its longitudinal length is controlled.

14. The system of claim 13, wherein at least a portion of the flexible tubular member corresponding to one or more adaptation points remains flexible.

15. The system of claim 1, wherein said first of the at least one lumen of the tubular member has a geometric cross section which minimises angular rotation of said at least one ablative element relative to said tubular member.

16. The system of claim 1, wherein said tubular member comprises a substantially flat bottom portion forming an active ablation side of said tubular member.

17. The system of claim 16, wherein said first of the at least one lumen of the tubular member has a substantially curvilinear surface oriented toward said substantially flat bottom portion and a substantially flat surface opposed to said substantially curvilinear surface.

## Patentansprüche

1. Ablationssystem mit einem undurchlässigen Bereich, das folgendes aufweist:
- ein Handhabungsteil (200) mit einem proximalen Ende und einem distalen Ende;
- ein flexibles langgestrecktes rohrförmiges Teil (122) mit mindestens einem sich hindurcherstreckenden Lumen (25), das betriebsmäßig an dem distalen Ende des Handhabungsteiles angebracht ist, wobei das flexible langgestreckte rohrförmige Teil aus einem porösen Material gebildet ist;
- eine Energiezuführungseinrichtung (27), die mindestens ein ablatives Element (26) aufweist, wobei die Energiezuführungseinrichtung betriebsmäßig an dem Handhabungsteil angebracht und dazu ausgelegt ist, sich innerhalb eines ersten Lumens von dem mindestens einen Lumen des rohrförmigen Teiles zu bewegen;
- eine Quelle für ablative Energie, die betriebsmäßig an dem mindestens einen ablativen Element angebracht ist; und
- ein Füllmaterial, das in Öffnungen (240) in der Nähe der äußeren Oberfläche des rohrförmigen Teiles (122) angeordnet ist, und zwar zumindest längs eines Bereiches des rohrförmigen Teiles, der einen Anfangsadaptionspunkt bildet,
wobei das flexible langgestreckte rohrförmige Teil an dem Adaptionspunkt für Fluide undurchlässig ist.

2. System nach Anspruch 1,
wobei der Anfangsadaptionspunkt die Länge des flexiblen rohrförmigen Teiles enthält, längs der sich die Energiezuführungseinrichtung bewegt.

3. System nach Anspruch 1,
wobei der Anfangsadaptionspunkt die gesamte Länge des flexiblen rohrförmigen Teiles enthält.

4. System nach Anspruch 2,
wobei das poröse Material expandiertes PTFE ist.

5. System nach Anspruch 3,
wobei das Füllmaterial ein Elastomer ist.

6. System nach Anspruch 4,
wobei das Füllmaterial Silikon ist.

7. System nach Anspruch 5,
wobei das mindestens eine ablative Element dazu ausgelegt ist, elektromagnetische Energie zu übertragen, und wobei das Füllmaterial für die übertragene elektromagnetische Energie transparent ist.

8. System nach Anspruch 2,
wobei das Füllmaterial flexibel ist, so daß die flexible Natur des rohrförmigen Teiles an dem Anfangsadaptionspunkt beibehalten wird.

9. System nach Anspruch 2,
wobei das Füllmaterial nicht flexibel ist, so daß die gesamte Flexibilität des rohrförmigen Teiles an dem Adaptionspunkt reduziert ist.

10. System nach Anspruch 2,
wobei das Füllmaterial steif ist, so daß die gesamte Flexibilität des rohrförmigen Teiles an dem Adaptionspunkt wesentlich reduziert ist.

11. System nach Anspruch 1,
wobei das Füllmaterial an einem oder mehreren Bereichen des rohrförmigen Teiles aufgebracht ist, die jeweils einen separaten Adaptionspunkt bilden.

12. System nach Anspruch 11,
wobei das Füllmaterial, das an jedem Adaptionspunkt verwendet wird, flexibel ist, so daß die gesamte Flexibilität des rohrförmigen Teiles flexibel bleibt.

13. System nach Anspruch 11,
wobei das Füllmaterial, das an jedem Adaptionspunkt verwendet wird, derart vorgegeben ist, daß der Wert der Flexibilität des rohrförmigen Teiles längs seiner longitudinalen Erstreckung gesteuert wird.

14. System nach Anspruch 13,
wobei zumindest ein Teil des flexiblen rohrförmigen Teiles, das einem oder mehreren Adaptionspunkten entspricht, flexibel bleibt.

15. System nach Anspruch 1,
wobei das erste Lumen von dem mindestens einen Lumen des rohrförmigen Teiles einen geometrischen Querschnitt besitzt, der eine winkelmäßige Drehung des mindestens einen ablativen Elementes relativ zu dem rohrförmigen Teil minimal macht.

16. System nach Anspruch 1,
wobei das rohrförmige Teil einen im wesentlichen flachen Bodenbereich aufweist, der eine aktive Ablationsseitde des rohrförmigen Teiles bildet.

17. System nach Anspruch 16,
wobei das erste Lumen von dem mindestens einen Lumen des rohrförmigen Teiles eine im wesentlichen krummlinige Oberfläche, die zu dem im wesentlichen flachen Bodenbereich orientiert ist, und eine im wesentlichen flache Oberfläche aufweist, die der im wesentlichen krummlinigen Oberfläche gegenüberliegt.

## Revendications

1. Système d'ablation avec une portion non perméable, comprenant :
une portion de poignée (200) comprenant des extrémités proximale et distale ;
un élément tubulaire flexible allongé (122) comprenant au moins un lumen (25) à travers lequel il est fixé de manière opérationnelle à l'extrémité distale de la portion de poignée, l'élément tubulaire flexible allongé étant constitué d'un matériau poreux ;
un dispositif d'alimentation en énergie (27) comprenant au moins un élément d'ablation (26), ce dispositif d'alimentation en énergie étant fixé de manière opérationnelle à la portion de poignée et étant conçu pour se déplacer à l'intérieur d'un premier lumen de l'élément tubulaire;
une source d'énergie d'ablation fixée de manière opérationnelle à au moins un élément d'ablation ; et
un matériau de remplissage disposé dans des ouvertures (240) à proximité de la surface externe de l'élément tubulaire (122) le long d'au moins une portion de l'élément tubulaire définissant un point d'adaptation initial,
dans lequel l'élément tubulaire flexible allongé est non perméable aux fluides au niveau du point d'adaptation.

2. Système de la revendication 1, dans lequel le point d'adaptation initial inclut la longueur de l'élément tubulaire flexible le long de laquelle le dispositif d'alimentation en énergie se déplace.

3. Système de la revendication 1, dans lequel le point d'adaptation initial inclut la longueur totale de l'élément tubulaire flexible.

4. Système de la revendication 2, dans lequel le matériau poreux est du PTFE expansé.

5. Système de la revendication 3, dans lequel le matériau de remplissage est un élastomère.

6. Système de la revendication 4, dans lequel le matériau de remplissage est du silicone.

7. Système de la revendication 5, dans lequel le (au moins un) élément d'ablation est conçu pour transmettre une énergie électromagnétique et le matériau de remplissage est transparent vis-à-vis de l'énergie électromagnétique transmise.

8. Système de la revendication 2, dans lequel le matériau de remplissage est flexible, moyennant quoi la nature flexible de l'élément tubulaire au niveau du point d'adaptation initial est conservée.

9. Système de la revendication 2, dans lequel le matériau de remplissage n'est pas flexible, moyennant quoi la flexibilité d'ensemble de l'élément tubulaire au niveau du point d'adaptation est réduite.

10. Système de la revendication 2, dans lequel le matériau de remplissage est rigide, moyennant quoi la flexibilité d'ensemble de l'élément tubulaire au niveau du point d'adaptation est considérablement réduite.

11. Système de la revendication 1, dans lequel le matériau de remplissage est déposé au niveau d'une ou plusieurs portions de l'élément tubulaire, chacune de ces portions définissant un point d'adaptation différent.

12. Système de la revendication 11, dans lequel le matériau de remplissage utilisé au niveau de chaque point d'adaptation est flexible, moyennant quoi la flexibilité d'ensemble de l'élément tubulaire est conservée.

13. Système de la revendication 11, dans lequel le matériau de remplissage utilisé à chaque point d'adaptation est prédéterminé de façon à ce que le degré de flexibilité de l'élément tubulaire le long de sa longueur longitudinale soit contrôlé.

14. Système de la revendication 13, dans lequel au moins une portion de l'élément tubulaire flexible, correspondant à un ou plusieurs points d'adaptation, reste flexible.

15. Système de la revendication 1, dans lequel ledit premier lumen de l'élément tubulaire présente une section transversale géométrique qui minimise la rotation angulaire dudit au moins un élément d'ablation par rapport audit élément tubulaire.

16. Système de la revendication 1, dans lequel ledit élément tubulaire comprend une portion inférieure sensiblement plate formant un côté d'ablation actif dudit élément tubulaire.

17. Système de la revendication 16, dans lequel ledit premier lumen de l'élément tubulaire présente une surface sensiblement incurvée, orientée vers ladite portion inférieure sensiblement plate, et une surface sensiblement plate opposée à ladite surface sensiblement incurvée.
